# FASCICULE DE BREVET EUROPEEN

(11) **EP 1 334 211 B1**
(45) Date de publication et mention de la délivrance du brevet: **15.03.2006**
(21) Numéro de dépôt: 01996626.6
(22) Date de dépôt: 16.11.2001
(51) Int. Cl.: C12Q 1/68

(54) **PROCEDE D'ANALYSE DE LA PREDISPOSITION GENETIQUE D'UN PATIENT AU DIABETE INSULINO-DEPENDANT, DISPOSITIF ET JEU D'AMORCES**
VEFAHREN ZUR ANALYSE DER GENETISCHEN PRÄDISPOSITION EINES PATIENTEN FÜR INSULINABHÄNGIGE DIABETES, TESTSATZ UND PRIMERSETS
METHOD FOR ANALYSING A PATIENT'S PREDISPOSITION TO INSULIN-DEPENDENT DIABETES, DEVICE AND SET OF PRIMERS

(30) Priorité: 17.11.2000 FR 0014896
(43) Date de publication de la demande: 13.08.2003
(73) Titulaire: Biomerieux S.A., 69280 Marcy l'Etoile (FR)
(72) Inventeur: MOUGIN, Bruno, F-69003 Lyon (FR)
(74) Mandataire: Bonneau, Gérard
(86) Numéro de dépôt international: PCT/FR2001/003599
(87) Numéro de publication internationale: WO 2002/040711

(56) Documents cités:
- WO-A-92/11389
- WO-A-98/28444
- WO-A-98/37237
- FR-A- 2 749 308
- SJOROOS M ET AL: "Triple-Label Hybridization Assay for Type-1 Diabetes-Related HLA Alleles." BIOTECHNIQUES, vol. 18, no. 5, 1995, pages 870-877, XP002177468 ISSN: 0736-6205
- HEZARD NATHALIE ET AL: "Factor V Leiden: Detection in whole blood by ASA PCR using an additional mismatch in antepenultimate position." THROMBOSIS RESEARCH, vol. 88, no. 1, 1997, pages 59-66, XP001024079 ISSN: 0049-3848 cité dans la demande
- TANG ET AL: "Primers and probes for HLA class II typing" THIRTEENTH INTERNATIONAL HISTOCOMPATIBILITY WORKSHOP, [en ligne] 4 avril 2000 (2000-04-04), XP002177469 Extrait de l'Internet: <URL:http://www.ihwg.org/protocols/ppclass ii3.pdf> [extrait le 2001-09-07]
- KOMULAINEN ET AL: "Clinical, autoimmune, and genetic characteristics of very young children with type 1" DIABETES CARE, vol. 22, no. 12, décembre 1999 (1999-12), page 1950-1955 XP002177470 cité dans la demande
- BODMER J G ET AL: "Nomenclature for factors of the HLA system, 1998." TISSUE ANTIGENS, vol. 53, no. 4 PART 2, avril 1999 (1999-04), pages 407-446, XP002177471 ISSN: 0001-2815
- PATEL P ET AL: "Rapid HLA typing by multiplex amplification refractory mutation system." JOURNAL OF CLINICAL PATHOLOGY (LONDON), vol. 46, no. 12, 1993, pages 1105-1108, XP001024211 ISSN: 0021-9746
- TISCH ROLAND ET AL: "Insulin-dependent diabetes mellitus." CELL, vol. 85, no. 3, 1996, pages 291-297, XP002177472 ISSN: 0092-8674
- NOBLE JANELLE A ET AL: "The role of HLA class II genes in insulin-dependent diabetes mellitus: Molecular analysis of 180 Caucasian, multiplex families." AMERICAN JOURNAL OF HUMAN GENETICS, vol. 59, no. 5, 1996, pages 1134-1148, XP001024212 ISSN: 0002-9297

### Liste de séquences

<110> bioMérieux S.A
<120> Procédé d'analyse de la prédisposition génétique d'un patient au diabète insulino-dépendant, dispositif adapté à sa mise en oeuvre et jeu d'amorces d'amplification adapté à un tel procédé
<130> HLADID
<140>
   <141>
<160> 14 4
<170> PatentIn Ver. 2.1 1
<210> 1
   <211> 21
   <212> ADN
   <213> Homo sapiens
<400> 1
   catgtgctac ttcaccaacg g 21
<210> 2
   <211> 21
   <212> ADN
   <213> Homo sapiens
<400> 2
   ctggtagttg tgtctgcaca c 21
<210> 3
   <211> 21
   <212> ADN
   <213> Homo sapiens
<400> 3
   cgcttcgaca gcgacgtggg g 21
<210> 4
   <211>20
   <212> ADN
   <213> Homo sapiens
<400> 4
   tatgaaactt atggggatac 20
<210> 5
   <211> 16
   <212> ADN
   <213> Homo sapiens
<400> 5
   tcttgtgagc agaagc 16
<210> 6
   <211> 14
   <212> ADN
   <213> Homo sapiens
<400> 6
   ccgcctgccg ccga 14
<210> 7
   <211> 16
   <212> ADN
   <213> Homo sapiens
<400> 7
   aggggacccg ggcgga 16
<210> 8
   <211> 16
   <212> ADN
   <213> Homo sapiens
<400> 8
   gacgtggagg tgtacc 16
<210> 9
   <211> 14
   <212> ADN
   <213> Homo sapiens
<400> 9
   gccgcctgac gccg 14
<210> 10
   <211> 14
   <212> ADN
   <213> Homo sapiens
<400> 10
   ggggcccggg cgtc 14
<210> 11
   <211> 14
   <212> ADN
   <213> Homo sapiens
<400> 11
   aggaggacgt gcgc 14
<210> 12
   <211> 16
   <212> ADN
   <213> Homo sapiens
<400> 12
   tcttgtaacc agatac 16
<210> 13
   <211> 18
   <212> ADN
   <213> Homo sapiens
<400> 13
   ggtggacacc gtatgcag 18
<210> 14
   <211> 17
   <212> ADN
   <213> Homo sapiens
<400> 14
   tggaacagcc agaagga 17

## Description

La présente invention concerne un procédé d'analyse de la prédisposition génétique d'un patient à au moins une maladie appelée le diabète insulino-dépendant.

Le diabète de type 1 (ou diabète insulino-dépendant) est une maladie caractérisée par la disparition des cellules β de Langerhans productrices d'insuline dans le pancréas, résultant d'un processus auto-immun qui se développe chez les individus génétiquement prédisposés. A côté de facteurs environnementaux, une composante génétique de prédisposition est observée.

Chaque individu dispose d'un patrimoine génétique propre, hérité de ses ascendants. Ce contexte génétique particulier peut parfois activement participer à l'apparition et/ou au développement de certaines affections : infections par un agent pathogène (virus du SIDA par exemple), maladies autoimmunes (maladies rhumatismales par exemple). Les gènes du Complexe Majeur d'Histocompatibilité (CMH) notamment les gènes codant pour les antigènes HLA (Human Leukocyte Antigens), jouent un rôle prépondérant dans le développement des pathologies autoimmunes :
- non spécifiques d'organes, par exemple articulaires comme la polyarthrite rhumatoïde (Lawrence, 1970 ; Stastny, 1978 ; Khan, 1979 ; Stastny, 1983 ; Gregersen, 1986 ; Gregersen, 1987; Stastny, 1988; Todd, 1988; Wordsworth, 1989; Nepom, 1989; Hiraiwa, 1990; Nepom, 1991) ou la spondylarthrite ankylosante (Brewerton, 1973 ; Schlosstein, 1973 ; Benjamin, 1990), ou
- spécifiques d'organes et plus précisément de glandes endocrines comme le pancréas associé au diabète insulino-dépendant (Komulainen, 1999 ; Kulmala, 2000).

Une partie importante de la composante génétique de la susceptibilité au diabète insulino-dépendant a pu être associée au marqueur principal lié au locus HLA permettent actuellement de considérer les allèles les plus courants parmi les quarante-cinq (45) allèles HLA-DQB1 décrits dans la nomenclature internationale, comme allèles de susceptibilité (allèles S), allèles de protection (allèles P), allèles neutres (allèles N) (www.anthonynolan.com/HIG/seq/nuc; octobre 2000). Il s'agit essentiellement de données validées pour des populations caucasiennes. Pour le moment, l'explication moléculaire réside dans le polymorphisme observé au niveau de l'acide aminé 57 de la chaîne β de la protéine HLA-DQ (Todd et al., 1988). Le tableau 1 ci-dessous résume le statut du diabète insulino-dépendant en fonction de la nature de cet acide aminé en position 57.

**Tableau 1 : Statut du diabète insulino-dépendant en fonction de la nature de l'acide aminé en position 57**

| Statut diabète insulino-dépendant | Allèles HLA-DQB1* | Acide aminé en position 57 |
|---|---|---|
| S | 02 | Alanine (A) |
| | 0302 | |
| | 0301 | |
| P | 0602 | Acide aspartique (D) |
| | 0603 | |
| N | Autres | Valine (V) ou Sérine (S) |

Chaque génotype correspond à une combinaison de deux allèles. Ainsi, à chaque échantillon correspond un génotype de prédisposition au diabète de type 1 : SS, PP, NN, SP, SN ou NP. Cette explication moléculaire est également cohérente avec l'observation d'une sévérité graduelle de la maladie lorsque le génotype comprend aucun, un ou deux allèles de susceptibilité, communément appelée effet dose.

*Actuellement en ce qui concerne le diabète insulino-dépendant, un article de Cinek et* al. *« Screening for the IDDM high-risk genotype. A rapid microtitre plate method using serum as source of DNA », Tissue Antigens, 2000, 56, 4, 344-349, décrit une méthode d'analyse. Cette publication souligne l'intérêt de développer un test d'analyse de prédisposition génétique au diabète insulino-dépendant. Cette équipe a choisi une approche à partir de sérum avec une technique en deux étapes qui est complète vis-à-vis des allèles concernés (quelques allèles HLA-DQB1 mais aussi quelques allèles HLA-DQA1 et quelques allèles HLA=DRB1*04).*

L'utilisation de sérum est beaucoup moins pratique qu'un protocole à partir d'une tâche de sang séché. De plus, leur technique est complexe puisque :
- ils réalisent une analyse sur plusieurs types d'allèles. Il s'agit de plus d'une analyse en deux temps des allèles HLA-DQB1 et HLA-DQA, puis des allèles HLA-DRB1*04,
- ils utilisent une technologie compliquée pour l'immobilisation des sondes de capture, avec une température d'hybridation à 63°C, des lavages à 63°C puis de 18 à 25°C et révélation en deux temps avec streptavidine péroxydase (SPOD).

De même, cette technique ne permet absolument pas de détecter l'effet dose qui a pourtant une influence considérable sur la prédisposition génétique d'un patient au diabète insulino-dépendant**.**

Enfin, ces tests ne peuvent être effectuées que par les centres spécialisés dans l'étude des gènes HLA, tels les centres de transfusions sanguines ou certains hôpitaux spécialisés. Cette identification nécessite donc l'envoi d'un échantillon et l'utilisation d'une technologie assez lourde. Les conséquences négatives sont donc nombreuses, telles que :
- les risques de perte de l'échantillon,
- la longue durée de cette identification,
- le coût relativement élevé de ladite identification, et
- l'absence de contrôle par le demandeur sur le prestataire de services.

Le procédé d'analyse revendiqué permet une analyse simplifiée sur le plan pratique, plus rapide, moins de deux heures, après préparation des amplicons, et peu onéreuse.

*Un article de SJOROOS M ET AL : **«** Triple-Label Hybridization Assay for Type-1 Diabetes-Related HLA Alleles. **»,** BIOTECHNIQUES, vol. 18, no. 5, 1995, pages 870-877. expose une méthode de détection d'allèles de type 1, deux allèles de susceptibilité (DQB1*0201 et DQB1*0302) et deux allèles de protection (DQB1*0602*/**0603 et DQB1*0301) pour le diabète insulino-dépendant. Pour cela, ils utilisent dans deux compartiments différents, deux sondes spécifiques des deux allèles de susceptibilité (WO243 et WO248), et deux sondes spécifiques des trois allèles de protection (WO329 et WO212). Aucune sonde ne permet de détecter toute ou partie des allèles neutres ou d'éventuels autres allèles de susceptibilité. Il n'est donc pas possible d'analyser la prédisposition d'un patient à une maladie auto-immune, telle que le diabète insulinodépendant.*
*De même, l'article de TISCH ROLAND ET AL : « Insulin-dependent diabets mellitus », CELL, vol. 85, no. 3, 1996, pages 291-297 divulgue l'utilisation de seulement quelques allèles de type DQB1, à savoir : trois allèles de susceptibilité, à savoir DQB1*0201, DQB1*0302, et DQB1*0502, et trois allèles de protection, à savoir DQB1*0301, DQB1*0303, et DQB1*0602. Ce document suggère une méthode de détection du diabète insulinodépendant qui en plus de la détection d'allèles DQB1 requière la détection d'allèles de type DRB1. Ce document ne permet donc pas non plus de détecter les allèles neutres ou d'éventuels autres allèles de susceptibilité et base ces méthodes de détection sur des allèles de type différent, à savoir HLA-DQB1 et HLA-DRB1, ce qui complexifie la méthode d'analyse .*
*L'article de TANG ET AL **«** Primers and probes for HLA class II typing » THIRTEENTH INTERNATIONAL HISTOCOMPATIBILITY WORKSHOP du 4 avril 2000, extrait de l'internet : <URL :http:*//*WWW.ihwg.org*/*protocols*/*ppclassii3.pdf>, correspond à un manuel décrivant l'ensemble des amorces et sondes pour le typage du HLA et notamment du HLA-DQB1, sans plus de précision. L'existence d'allèles de susceptibilité, de protection et de neutralité, le lien entre ces allèles et des maladies auto-immunes ne sont pas décrits. De plus, ce document base également les procédés d'analyse également sur la détection de types d'allèles autres que les allèles HLA-DQB1.*
*Le document WO 98 37237 A divulgue également une méthode de détection d'allèles HLA-DQB1, à savoir trois groupes d'allèles : les allèles de susceptibilité*
*(DQB1*0201*/**0202, et DQB1*0302*/**0303) et un allèle de protection (DQB1*0602) pour le diabète insulinodépendant. La méthode décrite dans ce document nécessite de plus la détection de l'allèle DRA. Le document WO 98 37237 A ne permet donc pas de détecter l'ensemble des allèles relatifs à l'insulinodépendance et décrit une méthode complexe.*
*Le document FR 2 749 308 A propose des sondes de typage du HLA DQB1, qui peuvent notamment servir comme indicateur de la sensibilité à certaines maladies, telles que le diabète insulinodépendant. Cependant ces sondes ne permettent pas de détecter une susceptibilité, une protection et une neutralité vis-à-vis d'une maladie auto-immune, et notamment du diabète insulinodépendant.*

A cet effet, la présente invention concerne un procédé d'analyse de la prédisposition génétique d'un patient à une maladie auto-immune, consistant à mettre un échantillon liquide contenant au moins un type d'amplicons, issus de l'amplification d'au moins une région polymorphe d'intérêt en rapport avec la maladie recherchée, à savoir le diabète insulinodépendant, en présence simultanément de sondes choisies de la façon suivante :
- au moins une sonde spécifique des allèles HLA-DQB1*0201 et HLA-DQB1*0202 et HLA-DQB1*0302 pour la susceptibilité,
- au moins une sonde spécifique des allèles HLA-DQB1*0301, HLA-DQB1*0602 et HLA-DQB1*0603 pour la protection; et
- au moins une sonde spécifique des autres allèles associés au diabète insulino-dépendant pour la neutralité.

Selon une variante de réalisation, les sondes utilisées pour détecter le diabète insulino-dépendant sont définies comme suit :
- au moins une sonde spécifique des allèles HLA-DQB1*0201, HLA-DQB1*0202, HLA-DQB1*0203, HLA-DQB1*0302, HLA-DQB1*0304, HLA-DQB1*0305, HLA-DQB1*0307 et HLA-DQB1*0308 pour la susceptibilité,
- au moins une sonde spécifique des allèles HLA-DQB1*03011, HLA-DQB1*03012, HLA-DQB1*03032, HLA-DQB1*03033, HLA-DQB1*0304, HLA-DQB1*0306, HLA-DQB1*0308, HLA-DQB1*0309, HLA-DQB1*0310, HLA-DQB1*0602, HLA-DQB1*0603, HLA-DQB1*0608, HLA-DQB1*0610, HLA-DQB1*06111, HLA-DQB1*06112, HLA-DQB1*0612, HLA-DQB1*0613, HLA-DQB1*0614 et HLA-DQB1*0616 pour la protection, et
- au moins une sonde spécifique des allèles HLA-DQB1*0306, HLA-DQB1*0401, HLA-DQB1*0402, HLA-DQB1*05011, HLA-DQB1*05012, HLA-DQB1*0502, HLA-DQB1*05031, HLA-DQB1*05032, HLA-DQB1*06011, HLA-DQB1*06012, HLA-DQB1*06013, HLA-DQB1*06051, HLA-DQB1*06052, HLA-DQB1*0606, HLA-DQB1*0609, HLA-DQB1*06112 et HLA-DQB1*0612 pour la neutralité.

Plus précisément et dans toutes les variantes précédemment citées, les sondes utilisées pour détecter la susceptibilité vis-à-vis du diabète insulino-dépendant sont définies comme suit:
- une sonde spécifique des allèles HLA-DQB1*0201, HLA-DQB1*0202 et HLA-DQB1*0203, et
- une sonde spécifique des allèles HLA-DQB1*0302, HLA-DQB1*0304, HLA-DQB1*0305, HLA-DQB1*0307 et HLA-DQB1*0308.

Selon ce dernier cas, les sondes utilisées pour détecter la susceptibilité vis-à-vis du diabète insulino-dépendant sont constituées d'au moins dix (10) nucléotides successifs pris dans les séquences suivantes :
- SEQ ID NO 5 (TCTTgTgAgCAgAAgC), et
- SEQ ID NO 6 (CCgCCTgCCgCCgA).

Plus précisément et dans toutes les variantes précédemment citées, les sondes utilisées pour détecter la protection vis-à-vis du diabète insulino-dépendant sont définies comme suit :
- une sonde spécifique des allèles HLA-DQB1*03011, HLA-DQB1*03012, HLA-DQB1*0304 et HLA-DQB 1*0309,
- une sonde spécifique des allèles HLA-DQB1*03011, HLA-DQB1*03012, HLA-DQB1*03032, HLA-DQB1*03033, HLA-DQB1*0306, HLA-DQB1*0309 et DQB1*0310, et
- une sonde spécifique des allèles HLA-DQB1*0308, HLA-DQB1*0602, HLA-DQB1*0603, HLA-DQB1*0608, HLA-DQB1*0610, HLA-DQB1*06111, HLA-DQB1*06112, HLA-DQB1*0612, HLA-DQB1*0613, HLA-DQB1*0614 et HLA-DQB1*0616.

Selon ce dernier cas, les sondes utilisées pour détecter la protection vis-à-vis du diabète insulino-dépendant sont constituées d'au moins dix (10) nucléotides successifs pris dans les séquences suivants :
- SEQ ID NO 7 (AggggACCCgggCggA),
- SEQ ID NO 8 (gACgTggAgTgTACC),et
- SEQ ID NO 9 (gCCgCCTgACgCCg).

Plus précisément et dans toutes les variantes précédemment citées, les sondes utilisées, pour détecter la neutralité vis-à-vis du diabète insulino-dépendant sont définies comme suit:
- une sonde spécifique des allèles HLA-DQB1*0306, HLA-DQB1*040 et HLA-DQB1*0402,
- une sonde spécifique des allèles HLA-DQB1*05011, HLA-DQB1*05012, HLA-DQB1*0502, HLA-DQB1*05031 et HLA-DQB1*05032,
- une sonde spécifique des allèles HLA-DQB1*06011, HLA-DQB1*06012 et HLA-DQB1*06013, et
- une sonde spécifique des allèles HLA-DQB1*06051, HLA-DQB1*06052, HLA-DQB1*0606, HLA-DQB1*0609, HLA-DQB1*06 112 et HLA-DQB1*0612.

Selon ce dernier cas, les sondes utilisées pour détecter la neutralité vis-à-vis du diabète insulino-dépendant sont constituées d'au moins dix (10) nucléotides successifs pris dans les séquences suivants : .
- SEQ ID NO 10 (ggggCCCgggCgTC).
- SEQ ID NO 11 (AggAggACgTgCgC),
- SEQ ID NO 12 (TCTTgTAACCAgATAC), et
- SEQ ID NO 13 (ggTggACACCgTATgCAg).

Dans tous les cas de figure, au moins une sonde de contrôle positif, capable de s'hybrider avec l'ensemble des gènes HLA-DQB1, est utilisée pour permettre la détection de tous les allèles HLA-DQB1.

De plus, au plus 38,89 %, préférentiellement au plus 20 %, des bases d'une même sonde sont remplacées par au moins une base analogue, telle que l'inosine. De telles valeurs sont déductibles d'une précédente demande de brevet déposée par la demanderesse sous le numéro PCT FR00/01385 sous priorité des 20 juin 1999 et 6 décembre 2000. Une définition d'une base analogue est donnée dans la suite de la description.

Préalablement au procédé d'analyse de la prédisposition génétique d'un patient à une maladie auto-immune, tel que décrit ci-dessus, au moins une amplification du gène HLA-DQB1 est effectuée.

Préférentiellement, les amorces d'amplification sont biotinylées, de sorte que les amplicons sont également biotinylés.

Préalablement à l'amplification, l'échantillon biologique prélevé, préférentiellement sous forme de tache de sang séché, est traité en vue de l'extraction des acides nucléiques.

Cette extraction des acides nucléiques s'effectue dans un mélange réactionnel contenant déjà les désoxyribonucléotides triphosphates (dNTP), qui seront ensuite utilisés lors de l'amplification, et ce préalablement à l'incubation.

Dans ce dernier cas, après l'incubation, on ajoute dans le mélange réactionnel, des désoxyribonucléotides triphosphates (dNTP), qui seront également utilisés lors de l'amplification ultérieure.

L'invention concerne également un dispositif permettant la mise en oeuvre d'un procédé tel que décrit ci-dessus, où selon une première variante de réalisation, chaque type de sondes DBQ1 spécifiques de la susceptibilité, de la protection de la neutralité est fixé dans un compartiment indépendant, tel qu'un puits d'une plaque de microtitration, indépendandamment des autres sondes. Selon une deuxième variante de réalisation, chaque type de sondes utilisées pour détecter la susceptibilité ou la protection vis-à-vis du diabète insulino-dépendant est fixé dans un compartiment indépendant, tel qu'un puits d'une plaque de microtitration, indépendamment des autres sondes utilisées pour détecter cette susceptibilité ou cette protection, et toute ou partie des différents types de sondes utilisées pour détecter la neutralité vis-à-vis du diabète insulino-dépendant est fixée dans au moins un puits d'une plaque de microtitration.

Il est également possible de fixer deux types de sondes spécifiques différentes dans un même compartiment indépendant, tel qu'un même puits d'une plaque de microtitration, sans interaction entre elles.

Selon une autre variante de réalisation, l'ensemble des types de sondes utilisées pour détecter la susceptibilité ou la protection ou la neutralité vis-à-vis du diabète insulino-dépendant est fixé dans un seul et même compartiment, tel qu'un puits d'une plaque de microtitration.

Dans le cas où un compartiment, tel qu'un même puits d'un plaque de microtitration, ne contient qu'un seul type de sondes utilisées pour détecter la susceptibilité ou la protection, le procédé de révélation des hybridations réalisées avec le dispositif s'effectue par une révélation colorimétrique non localisée mettant en jeu une réaction enzymatique. Par exemple, il peut s'agir d'une révélation des hybridations réalisées entre chaque type de sonde et les amplicons par la péroxydase.

Dans le cas où un compartiment, tel qu'un même puits d'un plaque de microtitration, contient au moins deux types de sondes utilisées pour détecter la susceptibilité ou la protection, le procédé de révélation des hybridations réalisées avec un dispositif s'effectue par une révélation localisée. Par exemple, il peut s'agir d'une révélation des hybridations réalisées entre chaque type de sonde et les amplicons par fluorescence ou radioactivité.

De plus, les sondes de capture permettant l'hybridation de séquences correspondant au gène HLA-DQB1, en vue de l'analyse de la prédisposition génétique d'un patient au diabète insulino-dépendant, sont fixées au fond d'un puits de plaque de microtitration ou sur une bille par l'intermédiaire d'un bras aminé ou de biotine situé(e) en position 5' des sondes.

Les exemples ci-joints sont donnés à titre d'exemple explicatif et n'ont aucun caractère limitatif. Ils permettront de mieux comprendre l'invention.

Les exemples ci-joints sont donnés à titre d'exemple explicatif et n'ont aucun caractère limitatif. Ils permettront de mieux comprendre l'invention.

La présente invention concerne un procédé de détection de maladies génétiques qui est rapide et d'un faible coût. Cette nouvelle technologie peut être utilisée avec toutes les maladies génétiques et particulièrement avec la polyarthrite rhumatoïde, la spondylarthrite ankylosante, le diabète insulino-dépendant et d'autres maladies auto-immunes, telles que les connectivites (lupus, sclérodermie, ...) également rencontrées lors de consultation de rhumatologie.

Il s'agit d'un procédé dédié à l'analyse de prédispositions génétiques d'un individu à certaines maladies, par une technique de biologie moléculaire permettant l'analyse simultanée d'au moins un gène. Ce procédé peut être avantageusement appliqué à l'analyse des prédispositions génétiques d'un individu, pour une maladie ou un ensemble de maladies apparentées, associée(s) à un ou plusieurs gènes. Ce procédé peut être appliqué à l'analyse des prédispositions génétiques d'un individu à certaines maladies auto-immunes, c'est-à-dire le diabète insulino-dépendant, relatif à un organe, dans le cas présent le pancréas.

L'intérêt de ce procédé est d'obtenir en une étape, avec un test multiple mais unique, un ensemble complet d'informations pertinentes d'intérêt clinique (diagnostique, pronostique et d'orientation thérapeutique). Le procédé se décompose en plusieurs étapes :
1) extraction d'acides nucléiques à partir d'un prélèvement biologique de l'individu,
2) amplification des régions d'intérêt, pour lesquelles un polymorphisme associé à une prédisposition génétique à une pathologie a été décrit, et
3) analyse simultanée des amplicons, utilisant un ensemble de réactions d'hybridation mettant en oeuvre un jeu de sondes moléculaires permettant l'analyse précise d'allèles ou de groupes d'allèles donnés.

### I -Définitions :

Par compartiment, il faut comprendre tout support solide plan ou concave, c'est-à-dire formant cuvette, permettant de recevoir soit :
- dans un premier cas, une quantité d'un liquide sans que ce liquide n'ait d'interaction avec d'autres aliquotes de ce liquide ou d'autres liquides présents au niveau d'éventuel(s) compartiment(s) adjacent(s),
- dans un second cas, plusieurs quantités égales ou différentes entre elles d'un même liquide ou de liquides différents sans que chacun de ce ou ces liquides n'ait d'interaction avec d'autres aliquotes de ce liquide ou d'autres liquides présents au niveau d'éventuel(s) compartiment(s) adjacent(s).

Un dispositif permettant de réaliser ces dépôts de quantités de liquide(s), qui peuvent aller de quelques centaines de microlitre, dans le premier cas, à 0,5 nanolitre, dans le second cas, ainsi que le procédé .mis en oeuvre par un tel dispositif et les supports réalisés avec le procédé par le dispositif sont déjà bien décrits dans une demande de brevet déposée le 15 novembre 2000 par la demanderesse, sous le numéro FR00/14691.

Dans le second cas, les différentes quantités de liquide(s) réparties sur un seul compartiment sont de très faibles volumes, généralement inférieurs au microlitre et forment des spots à la surface dudit compartiment. Le volume de solution par goutte est compris entre 0,5 nanolitre et 1 microlitre, préférentiellement 1 nanolitre et 200 nanolitres.

Le terme support solide, tel qu'utilisé dans la définition précédente, inclut tous les matériaux sur lesquels peut être immobilisé un analyte, et dans notre cas un acide nucléique. Des matériaux naturels, de synthèse, modifiés ou non chimiquement, peuvent être utilisés comme support solide, notamment des polymères tels que polychlorure de vinyle, polyéthylène, polystyrène, polyacrylate, polyamide, ou copolymères à base de monomères vinyl aromatiques, alkylesters d'acides α-insaturés ou β-insaturés, esters d'acides carboxyliques insaturés, chlorure de vinylidène, diènes ou composés présentant des fonctions nitriles (acrylonitrile); des polymères de chlorure de vinyle et de propylène, polymère de chlorure de vinyle et acétate de vinyle, copolymères à base de styrènes ou dérivés substitués du styrène ; des fibres synthétiques telles que le nylon, la nitrocellulose ; des matériaux inorganiques tels que la silice, le verre, la céramique, le quartz ; des latex ; des particules magnétiques ; des dérivés métalliques.

Le support solide selon l'invention peut être, sans limitation, sous la forme d'une plaque de microtitration, d'une feuille, d'un tube, d'un puits, de particules ou d'un support plan comme une pastille, généralement appelée « wafer », de silice ou silicium. Préférentiellement, au moins une partie du support est plan comme un wafer de silicium ou le fond d'un puits d'une plaque de microtitration. Préférentiellement, ce compartiment est constitué par un puits de plaque de microtitration.

Le support solide est hydrophile et/ou hydrophobe en fonction des applications et de la nature de la solution contenant le ou les analytes. Par exemple, des zones hydrophiles sont utilisées pour le dépôt, lesdites zones hydrophiles étant délimitées par des zones hydrophobes ce qui permet de mieux contrôler le diamètre des spots

Par révélation d'une hybridation, il faut comprendre que l'on utilise un polynucléotide marqué par un réactif de marquage. Par réactif de marquage, on entend un traceur générant directement ou indirectement un signal détectable. Une liste non limitative de ces traceurs suit :
- les enzymes, comme la péroxydase de raifort, la phosphatase alcaline, la β-galactosidase, la glucose-6-phosphate déshydrogénase, qui produisent un signal détectable en présence d'un substrat adapté, qui est ajouté, par exemple par colorimétrie, fluorescence, luminescence, ou
- les chromophores comme les composés fluorescents, luminescents, colorants, ou
- les groupements à densité électronique détectable par microscopie électronique ou par leur propriété électrique comme la conductivité, fampérométrie, la voltamétrie, les mesures d'impédance, ou
- les groupements détectables par des méthodes optiques comme la diffraction, la résonance plasmon de surface, la variation d'angle de contact ou physiques comme la spectroscopie de force atomique, l'effet tunnel, ou
- les molécules radioactives comme le ³²P, le ³⁵S ou le ¹²⁵I.

De préférence, le traceur est un composé fluorescent de faible encombrement stérique comme la fluorescéine, le dansyl, les chromophores du type IR (Li-COR Inc, Lincoln NE, USA), Cy5 et Cy3 (Randolph J.B. and al, Nucleic Acids Res., 25(14), p2923-2929, 1997) et leurs dérivés. Par faible encombrement stérique, on entend un poids moléculaire inférieur à 1000 g/mole.

Le procédé de détection d'un acide nucléique cible dans un échantillon dans lequel on met en contact cet acide nucléique cible, éventuellement prétraité, avec en outre le nucléotide fonctionnalisé de manière à générer un polynucléotide fonctionnalisé, et à marquer ledit polynucléotide avec le réactif de marquage puis à détecter ledit polynucléotide marqué.

Par prétraitement, on entend les différentes étapes de traitement de l'échantillon pour rendre accessible l'acide nucléique cible, comme par exemple, la lyse, la fluidification, la concentration.

Par «au moins une sonde spécifique des autres allèles associés au diabète insulino-dépendant pour la neutralité », il faut comprendre au moins une sonde spécifique de tout ou partie des allèles constituant le HLA-DQB1, allèles qui n'ont pas été définis comme constituant des allèles de susceptibilité ou de protection au diabète insulino-dépendant. Généralement, on choisit les sondes en fonction de la prévalence des allèles reconnus ; ainsi plus un allèle est prévalent et plus on l'implique au niveau des sondes spécifiques de neutralité.

Il peut également s'agir, seul ou en combinaison avec au moins l'une des sondes qui précèdent, d'au moins une sonde spécifique de tout ou partie des allèles constituant le HLA-DR3 et/ou le HLA-DR4. Ainsi de nombreux articles mettent en évidence ces haplotypes peuvent avoir un impact :
- Park Y.S., She J.X., Noble J.A., Erlich H.A. et Einsenbarth G.S., Tissue Antigens 2001 : 57: 185-191: « Transracial evidence for the influence of the homologous HLA DR-DQ haplotype on transmission of HLA DR4 haplotypes to diabetic children »,
- Kockum I., Sanjeevi C.B., Eastman S., Landin-Olsson M., Dahlquist G., Lernmark A. *et al.,* European Journal of Immunogenetics 26, 361-372 : « Complex interaction between HLA DR and DQ in conferring risk for childhood type 1 diabetes »,
- Undlien D.E., Kockum I., Ronningen K.S., Lowe R., Sanjeevi C.B., Graham J., Lie B.A., Akselsen H.E., Lernmark A. and Thorsby E., Tissue Antigens 1999 : 54, 543-551 : « HLA associations in type A diabetes among patients not carrying high-risk DR3-DQ2 or DR4-DQ8 haplotypes »,
- Donner H., Seidl C., Van der Auwera B., Braun J., Siegmund T., Herwig J., Weets I., The Belgian Diabetes Registry, Usadel K.H. et Badenhoop K., Tissue Antigens 2000 : 55 : 271-274 : « HLA-DRB1*04 and susceptibility to type 1 diabetes mellitus in a Gennan/Belgian family and German case-control study »,
- Redondo M.J., Kawasaki E., Mulgrew C.L., Noble J.A., Erlich H.A., Freed B.M., Lie B.A., Thorsby E., Einsenbarth G.S., Undlien D.E., Kockum I. et Ronningen K.S., The Journal of Clinical Endocrinology & Metabolism, 2000 : Vol.55, No 10 : 3793-3797 : « DR- and DQ-Associated Protection from Type 1A Diabètes: Comparison of DRB1*1401 and DQA1 *0102-DQB1*0602 ».

Par amplification, il faut comprendre que les polynucléotides fonctionnalisés sont générés par une réaction d'amplification enzymatique qui agit sur l'acide nucléique cible qui sert- de matrice. Les articles de Lewis (1992. Genetic Engineering News, 12, p. 1-9) d'une part, et d'Abramson et Myers (1993. Curr. Opin. Biotechnol., 4, p. 41-47), d'autre part, donnent des exemples d'amplification de cible. La technique d'amplification enzymatique est par exemple choisie parmi les techniques NASBA (Nucleic Acid Sequence Based Amplification), TMA (Transcription Mediated Amplification) RT-PCR (Reverse Transcriptase-Polymerase Chain Reaction), PCR (Polymerase Chain Reaction), SDA (Strand Displacement Amplification) ou LCR (Ligase Chain Reaction).

Par base analogue, il faut comprendre un nucléotide modifié qui est généralement incorporé dans un polynucléotide. Un polynucléotide est constitué d'un enchaînement d'au moins deux désoxyribonucléotides ou ribonucléotides comprenant éventuellement au moins un nucléotide modifié, par exemple au moins un nucléotide comportant une base modifiée, tel que l'inosine, la méthyl-5-désoxycytidine, la diméthylamino-5-désoxyuridine, la désoxyuridine, la diamino-2,6-purine, la bromo-5-désoxyuridine, la nébularine ou toute autre base modifiée permettant l'hybridation. Ce polynucléotide peut aussi être modifié au niveau :
- de la liaison intemucléotidique, comme par exemple les phosphorothioates, les H-phosphonates, les alkyl-phosphonates, ou
- du squelette, comme par exemple les α-oligonucléotides (FR-A-2.607.507) ou les PNA (M. Egholm et al., J. Am. Chem. Soc., 114, p1895-1897, 1992 ou les 2' O-alkyl ribose.

Chacune de ces modifications peut être prise en combinaison. Le polynucléotide peut être un oligonucléotide, un acide nucléique naturel ou un de ses fragments comme un ADN, un ARN ribosomique, un ARN messager, un ARN de transfert, un acide nucléique obtenu par une technique d'amplification enzymatique.

### II - Préparations de l'échantillon :

### 1°) Extraction d'acides désoxyribonucléiques (ADN) :

### A - A partir d'un échantillon de sang total:

Les différents protocoles classiques d'extraction d'ADN à partir d'échantillons de sang total prélevé sur un anticoagulant, comme EDTA (Ethylène diamine tétraacétique) ou citrate ou héparine, peuvent être utilisés. Ainsi, il peut s'agir d'un protocole avec une étape d'extraction au phénol ou avec étapes de lyses des cellules rouges puis des cellules blanches (Kimura *et al.,* 1992).

En pratique et selon un mode de réalisation, cette extraction est réalisée de manière tout à fait classique. En fait, on peut utiliser n'importe quelle technique d'extraction d'ADN, permettant d'obtenir du matériel susceptible d'être ultérieurement amplifié par un procédé d'amplification comme la Polymerase Chain Reaction (PCR). Ces techniques de lyse des cellules, avec extraction puis purification des acides nucléiques sont habituellement celles recommandées pour des analyses génétiques ou des techniques rapides utilisant des produits commerciaux, telles que QIAmp Blood Kit (Marque déposée) de QIAGEN S.A.

### B - A partir de taches de sang séché :

Différents protocoles d'extraction d'ADN à partir de sang séché sont décrits dans la littérature.

Il y a tout d'abord une première **technique de Jinks et *al*.,** Hum. Genet. 1989, 81 : 363-366. Celle-ci consiste à déposer du sang sur du papier Schleicher & Schuell no. 903. Les taches sont séchées à une température comprise entre 18 et 25°C, pendant plusieurs heures. On découpe quatre pastilles de 3 mm de diamètre, et on les place dans un tube de 1,5 ml et on fixe par addition de 30 µl de méthanol, puis on effectue une évaporation. Ensuite on ajoute 60 µl d'eau et on porte à ébullition pendant 15 minutes. Après on centrifuge 15 minutes à 10.000 g. Enfin on prélève le surnageant pour l'étape suivante, l'amplification par PCR.

La deuxième **technique** est celle **de Hezard et** ***al.,*** Thrombosis Research, 1997, 88, 1, 59-66. On collecte le sang sur. EDTA ou sur citrate ou sur héparine, et on le dépose sur un papier filtre (Guthrie). Une pastille de 1 mm de diamètre est directement placée dans 50 µl de mélange réactionnel pour l'amplification subséquente. On incube 15 minutes à 94°C. Et enfin on additionne de la Taq polymérase, puis on amplifie par PCR.

Enfin, depuis cette année 2000, la **société Molecular Innovations Inc.** commercialise un système d'extraction d'ADN, qui constitue la troisième **technique,** situé à l'intérieur même du tube d'amplification (Xtra Amp (marque déposée) Extraction System, Molecular Innovations Inc. Réf. 660). Nous avons évalué ce produit, en vue d'extraire l'ADN à partir de tache de sang. Le sang est collecté sur EDTA ou ACD, déposé sur papier Schleicher & Schuell réf. 322187. Une pastille de 3 mm de diamètre est placée dans un microtube de 1,5 ml contenant 75 µl de Lyis Buffer. On incube 10 minutes à une température comprise entre 18 et 25°C. Puis, on centrifuge 5 minutes à 12.000 g. On prélève ensuite le surnageant et on le transfère dans un tube Xtra Amp (marque déposée). On aspire et refoule plusieurs fois puis on élimine le surnageant. On lave trois fois avec 200 µl de Wash Buffer. Après on ajoute 45 µl de mélange réactionnel pour l'amplification PCR et 5 µl de Amp Enhance Buffer. Enfin on procède à l'amplification en ajoutant trois cycles au programme d'amplification habituel.

Avec les trois protocoles décrits ci-dessus, nous avons observé une amplification du HLA-DQB1, avec de bons résultats exploitables pour le test ELOSA (Enzyme Linked OligoSorbent Assay) mis au point selon la présente invention, dans environ 50% des cas. Cette technique ELOSA est bien décrite dans le brevet EP-B-0.486.661 de la demanderesse ou dans l'article de F. Mallet et *al.,* Journal of Clinical Microbiology, June 1993, p.1444-1449.

Néanmoins, il convient de développer un protocole d'extraction à partir de taches de sang, protocole qui soit robuste et indispensable pour les applications d'analyse en routine. Une quatrième **technique** a donc été développé **par la demanderesse** qui consiste à :
- collecter le sang sur EDTA, déposé sur papier Schleicher & Schuell no. 903 (réf. 322187),
- découper la tache de sang à l'aide de la poinçonneuse de Schleicher & Schuell (pastille de 3 mm de diamètre), .
- décontaminer la poinçonneuse à l'aide d'HCl 0,25 N pendant 5 minutes (dépurination de l'ADN), après chaque tache de sang,
- placer la pastille dans un tube PCR de 0.5 ml,
- ajouter 50 µl d'un mélange réactionnel contenant 5 µl de tampon d'amplification dix fois concentré (10 X), 0,5 µl de mélange de dNTP (200 mM) et H₂O qsp 50µl,
- incuber 15 minutes à 100°C (recommandé d'utiliser un thermocycleur),
- retirer la pastille,
- centrifuger brièvement à 1.000 g par une centrifugeuse de paillasse classique, et
- prélever 25 µl de surnageant pour la réaction d'amplification.

De manière surprenante, le milieu réactionnel qui vient reprendre le sang présent sur chaque pastille contient les désoxyribonucléotides triphosphates (dNTP), c'est-à-dire les dATP, dCTP, dGTP et dTTP. Il s'agit de molécules biologiques que l'homme du métier cherche plutôt à protéger de températures élevées. Or il s'avère que dans le protocole mis au point par la demanderesse, ledit milieu réactionnel subit ensuite une température de 100°C pendant 15 minutes. Les résultats d'amplification qui sont obtenus par la suite avec les acides nucléiques extraits selon notre technique sont beaucoup plus efficaces que ceux obtenus avec les trois première techniques exposées.

### 2°) Préparation des amplicons HLA-DQB1 :

Dans tous les cas de figure, les amorces utilisées sont des amorces décrites dans la littérature (XIth HLA Workshop Primers ; Kimura, 1992). Le tableau 2 ci-dessous expose les deux amorces qui permettent l'amplification du locus d'intérêt correspondant au gène HLA-DQB1. Ces deux amorces encadrent donc ce gène.

**Tableau 2 : Amorces pour amplifier le gène HLA-DQB1**

| N° de séquence | Séquence (5' > 3') | Appellation scientifique |
|---|---|---|
| SEQ ID NO 1 | CATgTgCTACTTCACCAACgg | DQBAMP-A |
| SEQ ID NO 2 | CTggTAgTTgTgTCTgCACAC | DQBAMP-B |

### A - A partir d'un échantillon de sang total :

Le mélange réactionnel contient les différents constituants suivants :
- tampon 10X (Perkin Elmer, réf. N 808-0171) 5 µl,
- dNTPs 200 mM (Pharmacia, réf. 27-2094 0,5 µl (0,2 mM final),
- mélange d'amorces (30µM de chaque) 0,5 µl (0,3 µM final),
- Taq Polymerase(AmpliTaq, Perkin Elmer, réf. N 808-0171, 5U/µl) 0,3 µl (1,5 U),
- ADN (environ 100 ng/µl) 5 µl (environ 500 ng), et
- H₂O q.s.p. 50 µl.

De plus, le programme d'amplification est le suivant :
- 2 minutes à 95 °C, puis
- 35 cycles successifs avec pour chaque cycle les étapes suivantes :
   ■ 30 secondes à 95 °C,
   ■ 30 secondes à 55 °C, et
   ■ 30 secondes à 72 °C,
- 7 minutes à 72 °C.

Ensuite, si l'on veut conserver le produit de réaction, on maintient le tube contenant les amplicons qui viennent d'être obtenus à la température de 9°C.

### B - A partir de taches de sang séché:

Le mélange réactionnel contient les différents constituants suivants :
- tampon 10X (Perkin Elmer, réf. 27-2094) 5 µl,
- dNTPs 200 mM (Pharmacia, réf. N 808-0171) 0,5 µl,
- mélange d'amorces (30 µM de chaque) 0,5 µl (0,3 µM final),
- Taq Polymerase (AmpliTaq, Perkin Elmer, réf. N 808-0171, SU/µl) 0,3 µl (1,5 U),
- ADN 25 µl, et
- H₂O q.s.p. 50 µl.

De plus, le programme d'amplification est le suivant :
- 2 minutes à 95 °C, puis
- 35 cycles successifs avec pour chaque cycle les étapes suivantes :
   ■ 30 secondes à 95 °C,
   ■ 30 secondes à 55 °C, et
   ■ 30 secondes à 72 °C,
- 7 minutes à 72 °C.

Ensuite, si l'on veut conserver le produit de réaction, on maintient le tube contenant les amplicons qui viennent d'être obtenus à la température de 9°C.

### III - Analyse des amplicons obtenus selon l'invention :

### 1°) Dispositif d'analyse de la prédisposition génétique d'un patient au diabète insulino-dépendant :

Un dispositif basé sur le principe du test ELOSA a été réalisé pour l'analyse du marqueur HLA-DQB1, sous la forme d'une barrette de huit puits tirée d'une plaque de microtitration, avec une sonde de détection couplée à la péroxydase (POD) pour la révélation colorimétrique.

### A - Sondes de capture :

Dans chacun de ces huit puits des sondes spécifiques de capture ont été utilisées. Ces sondes sont en fait des oligonucléotides synthétisés avec un bras aminé en 5', telles que décrites dans les brevets US-A-5,510,084 et EP-B-0.549.776 déposés par la Demanderesse. C'est ce bras aminé en 5', de structure particulière, qui autorise la fixation des sondes en fond de plaque. Il faut donc comprendre que l'extrémité 5' des sondes utilisées possèdent ce bras, même s'il n'est pas précisé par la suite.

La répartition des sondes de capture au niveau des puits est précisée en tableau 3, alors que les allèles cibles possibles pour chaque sonde de capture du tableau 3 sont précisés dans le tableau 4 ci-dessous.

**Tableau 3 : Répartition des sondes de capture au niveau des puits**

| N° de séquence | Séquence (5' > 3') | Appellation bioMérieux |
|---|---|---|
| SEQ ID NO 3 | CgCTTCgACAgCgACgTgggg | C+ |
| SEQ ID NO 4 | TATgAAACTTATggggATAC | C- |
| SEQ ID NO 5 | TCTTgTgAgCAgAAgC | 26c |
| SEQ ID NO 6 | CCgCCTgCCgCCgA | 57f |
| SEQ ID NO 7 | AggggACCCgggCggA | 70b |
| SEQ ID NO 8 | gACgTggAggTgTACC | 45a |
| SEQ ID NO 9 | gCCgCCTgACgCCg | 57e |
| SEQ ID NO 10 | ggggCCCgggCgTC | 70a |
| SEQ ID NO 11 | AggAggACgTgCgC | 37b |
| SEQ ID NO 12 | TCTTgTAACCAgATAC | 26b |
| SEQ ID NO 13 | ggTggACACCgTATgCAg | 70c |

**Tableau 4 : Allèles cibles possibles pour chaque sonde de capture**

| N° de séquence | Spécificité HLA-DQB1* | Sensibilité / maladie |
|---|---|---|
| SEQ ID NO 3 | Tous | Néant |
| SEQ ID NO 4 | Aucun | Néant |
| SEQ ID NO 5 | **0201, 0202,** 0203 | Susceptibilité |
| SEQ ID NO 6 | **0302,** 0304, 0305, 0307, 0308 | Susceptibilité |
| SEQ ID NO 7 | **0602,0603,**0608,0610,06111,06112, 0612, 0613, 0614, 0616, 0308 | Protection |
| SEQ ID NO 8 | **03011, 03012,** 0304, 0309 | Protection |
| SEQ ID NO 9 | **03011, 03012,** 03032, 03033, 0306, 0309,0310 | Protection |
| SEQ ID NO 10 | 05011, 05012, 0502, 05031, 05032 | Neutralité |
| SEQ ID NO 11 | 06011,06012,06013 | Neutralité |
| SEQ ID NO 12 | 06051, 06052, 0606, 0609, 06112, 0612 | Neutralité |
| SEQ ID NO 13 | 0306, 0401, 0402 | Neutralité |

Dans le tableau 3, le puits C+ comporte un contrôle positif (SEQ ID NO 3), qui autorise la détection de tous les allèles du gène HLA-DQB1*, ce qui permet de contrôler que l'amplification a bien concerné la région d'intérêt comprise entre les amorces SEQ ID NO 1 et SEQ ID NO 2 décrites dans le tableau 1. Le contrôle négatif (SEQ ID NO 4) n'a pas d'objectif diagnostic, il n'est présent que pour répondre à certaines normes. Cette séquence n'est absolument pas spécifique du HLA, et correspond à une séquence aléatoire non retrouvée chez les gènes HLA.

Les autres sondes, comprises entre SEQ ID NO 5 et SEQ ID NO 13, ont par contre un but diagnostique de la prédisposition génétique d'un patient à une maladie, et plus précisément au diabète insulino-dépendant.

On remarque que la sonde SEQ ID NO 5 est une sonde qui détecte une susceptibilité à la maladie, c'est-à-dire au diabète insülino-dépendant. Elle est spécifique des allèles importants de cette susceptibilité à cette maladie, à savoir les allèles HLA-DQB1*0201 et HLA-DQB1*0202. Ces allèles importants sont représentés en gras dans le tableau 4.

De même, la sonde SEQ ID NO 6 est une sonde qui détecte également une susceptibilité au diabète insulino-dépendant. Elle est spécifique d'un allèle important de cette susceptibilité à cette maladie, à savoir l'allèle HLA-DQB1*0302.

La sonde SEQ ID NO 7 est une sonde qui détecte une protection contre la maladie, c'est-à-dire au diabète insulino-dépendant. Elle est spécifique des allèles importants de cette protection à cette maladie, à savoir les allèles HLA-DQB1*0602 et HLA-DQB1*0603.

De même, la sonde SEQ ID NO 8 est une sonde qui détecte également une protection contre le diabète insulino-dépendant. Elle est spécifique d'un allèle important de cette protection à cette maladie; à savoir l'allèle HLA-DQB1*0301, que celui-ci soit sous sa forme HLA-DQB1*03011 et HLA-DQB1*03012.

La sonde SEQ ID NO 9 est une sonde qui détecte aussi une protection contre le diabète insulino-dépendant, puisqu'elle permet également de détecter l'allèle HLA-DQB1*0301, que celui-ci soit sous sa forme HLA-DQB1*03011 ou HLA-DQB1*03012. L'intérêt de cette sonde est de permettre la détection de l'allèle HLA-DQB1*03032, qui est assez fréquent et doit être protecteur au regard de sa séquence au niveau de la position 57.

A noter que les deux sondes SEQ ID NO 6 et SEQ ID NO 7, l'une de susceptibilité et l'autre de protection vis-à-vis du diabète insulino-dépendant, permettent de détecter l'allèle HLA-DQB1*0308. Il convient de préciser que cet allèle est très rare. Les chances de le trouver sont donc infimes et les chances de tomber sur un homozygote HLA-DQB1*0308 et HLA-DQB1*0308 sont quasi nulles. De même on peut appliquer le même raisonnement pour les deux sondes SEQ ID NO 6 et SEQ ID NO 8, l'une de susceptibilité et l'autre de protection vis-à-vis du diabète insulino-dépendant. Elles permettent toutes les deux de détecter l'allèle HLA-DQB1*0304. Cet allèle est également très rare.

Dans ces deux cas, il n'y a aucun problème à ce que deux sondes, l'une de susceptibilité et l'autre de protection, puissent les détecter, car la prévalence est trop faible.

### B - Sondes de détection :

Il s'agit d'un oligonucléotide avec bras aminé en 5', couplée à la péroxydase (POD). Le tableau 5 précise sa structure.

**Tableau 5 : Sonde de détection adaptée aux sondes de capture utilisées**

| N° de séquence | Séquence (5' > 3') | Appellation bioMérieux |
|---|---|---|
| SEQ ID NO 14 | TggAACAgCCAgAAggA | D4-POD |

Bien entendu cette sonde de détection est choisie pour être complémentaire de l'ensemble des amplicons cibles pouvant être accrochés par les sondes de capture SEQ ID NO 3 et SEQ ID NO 5 à SEQ IDNO 13 présentées aux tableaux 3 et 4.

### C - Mode opératoire :

Premièrement, la **préparation des cibles** consiste à :
- transférer la totalité des amplicons (50 µl) dans un microtube de 1,5 ml,
- ajouter 5 µl de réactif R2 (NaOH 2N),
- bien homogénéiser,
- incuber pendant 5 minutes à une température comprise entre 18 à 25°C,
- ajouter 1 ml de tampon d'hybridation, dont la constitution est précisée ci-après,
- ajouter 50 µl de sonde de détection, dont la constitution est précisée ci-après, et
- bien homogénéiser.

Le tampon d'hybridation est constitué de :
- 0,1 M phosphate de sodium pH 6,8,
- 0,5 M NaCl, 2% (p/v) polyethylene glycol 4000,
- 0,65% (p/v) tween 20, 0,1 % (p/v) gélatine,
- 0,14 g/L d'ADN soniqué de sperme de saumon,
- 0,2 g/L de BND (biocide ou Bromo-Nitro-Dioxane), et
- 0,01 g/L de ciprofloxacine.

La sonde de détection SEQ ID NO 14 (D4-POD) est diluée en tampon phosphate 5 mM à un pH de 7,0, en présence de 0,5% (p/v) albumine sérique bovine et 0,5% (p/v) phénol.

Deuxièmement, l'**hybridation des cibles** est réalisée de la manière suivante :
- répartir 100 µl du mélange, dans chacun des huit puits de la barrette R1,
- recouvrir d'une feuille autocollante, et
- incuber pendant 1 heure à 37°C (37 ± 1°C).

Troisièmement, le **lavage** qui permet d'éliminer les cibles éventuelles qui ne se sont pas hybridées, et consiste à laver trois fois avec 500 µl de tampon de lavage Color 0, dilué au 1/20 en H₂O. Par « Color 0 », il faut comprendre une solution vingt (20) fois concentré de PBS, auquel on a ajouté 1 % (p/v) tween 20.

Quatrièmement, la **révélation** a pour objet de déterminer quelles sondes de capture ont hybridées un oligonucléotide cible. Elle est réalisée de la façon suivante :
- ajouter 100 µl de solution de substrat préparé extemporanément - 1 pastille de Color 1 dans 5 ml de Color 2, dans chacun des puits,
- incuber pendant 20 minutes à une température de 18 à 25°C, à l'obscurité,
- ajouter 50 µl de Color 3, et
- lire l'absorbance à 492 nm.

Par « Color 1 », il faut comprendre O-phenylenediamine dihydrochloride (OPD) ; par « Color 2 », il faut comprendre 0,1 M phosphate de sodium, 0,05 M acide citrique, 0,03% H₂O₂, et par « Color 3 », il faut comprendre 1,8 N H₂SO₄.

Cinquièmement, l'**interprétation** permet en fonction la révélation qui a été établie au chapitre précédent de déterminer la réelle prédisposition génétique d'un patient au diabète insulino-dépendant. Cette vérification propose de :
- d'une part, vérifier la positivité du contrôle positif SEQ ID NO 3 (C+), où la Densité Optique (D.O.) doit être supérieure à 0,8, et vérifier la négativité du contrôle négatif SEQ ID NO 4 (C-), où la D.O. doit être inférieure à 0,05),
- d'autre part, déterminer le génotype correspondant aux deux allèles identifiés à l'aide des sondes SEQ ID NO 5 à SEQ ID NO 13.

Dans le cas cité ci-dessus, la péroxydase est une enzyme qui permet de révéler pour chaque sonde si l'hybridation avec un oligonucléotide s'est réalisée si elle est en présence d'un substrat adéquat, l'OPD par exemple. Du fait que ce substrat est soluble, il y aura extension du signal. Dans ce cas, il faut, pour pouvoir déterminer si un patient est homozygote ou hétérozygote ou si ses allèles sont susceptibles, protecteurs et/ou neutre vis-à-vis du diabète insulino-dépendant, implanter un sonde de capture par compartiment ou, de manière plus aisée, par puits. Ceci est vrai pour les sondes concernées par la susceptibilité (SEQ ID NO 5 et 6) ou par la protection (SEQ ID NO 7, 8 et 9) vis-à-vis du diabète insulino-dépendant. Alors que pour les sondes concernées par la neutralité (SEQ ID NO 10, 11, 12 et 13) vis-à-vis de cette maladie, il est tout à fait possible de les regrouper.

Ceci n'est pas vrai si le substrat utilisé est précipitant ou si la sonde de détection (SEQ ID NO 14) est fluorescente. Dans ces cas, plusieurs sondes peuvent être implantées dans un compartiment ou un puits identique, même si ce sont des sondes de susceptibilité (SEQ ID NO 5 et 6) et/ou de protection (SEQ ID NO 7, 8 et 9). En fait, dans sa configuration la plus compacte, toutes les sondes de susceptibilité, de protection et de neutralité peuvent être dans le même compartiment ou puits, cela ne dépend que de la facilité avec laquelle l'opérateur est capable de grouper les différents types de sondes, sans qu'il y ait de chevauchement entre les plots d'oligonucléotides de capture. Comme précisé précédemment, un tel dispositif permettant de réaliser de tels supports est déjà bien décrit dans une demande de brevet déposée le 15 novembre 2000 par la demanderesse, sous le numéro FR00/14691.

### 2°) Exemples d'analyses selon l'invention :

### A - Premier échantillon :

Le tableau 6 qui suit présente les résultats obtenus avec une barrette R1 où chaque puits a reçu un aliquote d'un échantillon d'un premier patient à tester.

**Tableau 6 : Résultats obtenus avec la barrette R1 pour le premier échantillon**

| Barrette R1 | D. O. x 1000 | + /- |
|---|---|---|
| SEQ ID NO 3 (C+) | > 2500 | + |
| SEQ ID NO 4 (C-) | 0 | - |
| SEQ ID NO 5' (S) | 1245 | + |
| SEQ ID NO 6 (S) | 126 | + |
| SEQ ID NO 7 (P) | 7 | - |
| SEQ ID NO 8 (P) | 9 | - |
| SEQ ID NO 9 (P) | 1 | - |
| SEQ ID NO 10 à 13 (N) | 8 | - |

L'analyse est la suivante :
- la sonde SEQ ID NO 3 est positive : l'amplification du gène HLA-DQB1 et le test d'hybridation ont correctement fonctionné,
- la sonde SEQ ID NO 5 est positive : un allèle HLA-DQB1*0201 ou HLA-DQB1*0202 ou HLA-DQB1*0203 est présent,
- la sonde SEQ ID NO 6 est positive : un allèle HLA-DQB1*0302 ou HLA-DQB1*0304 ou HLA-DQB1*0305 ou HLA-DQB1*0307 ou HLA-DQB1*0308 est présent, et
- les autres sondes sont négatives.

En conclusion, il y a de forte chance que ce patient ait deux allèles de susceptibilité vis-à-vis du diabète insulino-dépendant. Par forte chance, il faut comprendre que la prévalence de l'allèle HLA-DQB1*0302 de susceptibilité est bien plus importante que celle des allèles HLA-DQB1*0304 ou HLA-DQB1*0305 ou HLA-DQB1*0307 ou HLA-DQB1*0308, qui sont des allèles de neutralité.

Ces deux allèles HLA-DQB1 ont été identifiés au niveau générique, le typage HLA de ce premier échantillon est en fait :
- HLA-DQB1*02, et
- HLA-DQB1*0302.

### B - Deuxième échantillon :

Le tableau 7 qui suit présente les résultats obtenus avec une barrette R1 où chaque puits a reçu un aliquote d'un échantillon d'un deuxième patient à tester.

**Tableau 7 : Résultats obtenus avec la barrette R1 pour le deuxième échantillon**

| Barrette R1 | D. O. x 1000 | + / - |
|---|---|---|
| SEQ ID NO 3 (C+) | 1685 | + |
| SEQ ID NO 4 (C-) | 5 | - |
| SEQ ID NO 5 (S) | 708 | + |
| SEQ ID NO 6 (S) | 22 | - |
| SEQ ID NO 7 (P) | 7 | - |
| SEQ ID NO 8 (P) | 143 | + |
| SEQ ID NO 9 (P) | 41 | - |
| SEQ ID NO 10 à 13 (N) | 11 | - |

L'analyse est la suivante :
- la sonde SEQ ID NO 3 est positive : l'amplification du gène HLA-DQB1 et le test d'hybridation ont correctement fonctionné,
- la sonde SEQ ID NO 5 est positive : un allèle HLA-DQB1*0201 ou HLA-DQB1*0202 ou HLA-DQB1*0203 est présent,
- la sonde SEQ ID NO 8 est positive : un allèle HLA-DQB1*03011 ou HLA-DQB1*03012 ou HLA-DQB1*0304 ou HLA-DQB1*0309 est présent, et
- les autres sondes sont négatives.

En conclusion, il y a de forte chance que ce patient ait un allèle de susceptibilité et un allèle de protection vis-à-vis du diabète insulino-dépendant. Par forte chance, il faut comprendre que la prévalence des allèles HLA-DQB1*03011 et HLA-DQB1*03012 de susceptibilité est bien plus importante que celle des allèles HLA-DQB1*0304 ou HLA-DQB1*0309, qui sont des allèles de neutralité.

Ces deux allèles HLA-DQB1 ont été identifiés au niveau générique, le typage HLA de ce premier échantillon est en fait :
- HLA-DQB1*02, et
- HLA-DQB1*0301.

### C - Troisième échantillon :

Le tableau 8 qui suit présente les résultats obtenus avec une barrette R1 où chaque puits a reçu un aliquote d'un échantillon d'un troisième patient à tester.

**Tableau 8 : Résultats obtenus avec la barrette R1 pour le troisième échantillon**

| Barrette R1 | D. O. x 1000 | +/- |
|---|---|---|
| SEQ ID NO 3 (C+) | >2500 | + |
| SEQ ID NO 4 (C-) | 0 | - |
| SEQ ID NO 5 (S) | 1790 | + |
| SEQ ID NO 6 (S) | 27 | - |
| SEQ ID NO 7 (P) | 31 | - |
| SEQ ID NO 8 (P) | 19 | - |
| SEQ ID NO 9 (P) | 15 | - |
| SEQ ID NO 10 à 13 (N) | 829 | + |

L'analyse est la suivante :
- la sonde SEQ ID NO 3 est positive :l'amplification du gène HLA-DQB1 et le test d'hybridation ont correctement fonctionné,
- la sonde SEQ ID NO 5 est positive : un allèle HLA-DQB1*0201 ou HLA-DQB1*0202 ou HLA-DQB1*0203 est présent,
- les sondes SEQ ID NO 10 à 13 sont positives : un allèle HLA-DQB1*0306 ou HLA-DQB1*0401 ou HLA-DQB1*0402 ou HLA-DQB1*05011 ou HLA-DQB01*05012 ou HLA-DQB1*0502 ou HLA-DQB1*05031 ou HLA-DQB1*05032 ou HLA-DQB1*06011 ou HLA-DQB1*06012 ou HLA-DQB1*06013 ou HLA-DQB1*06051 ou HLA-DQB1*06052 ou HLA-DQB1*0606 ou HLA-DQB1*0609 ou HLA-DQB1*06112 ou HLA-DQB1*0612 est présent, et
- les autres sondes sont négatives.

En conclusion, il est certain que ce patient a un allèle de susceptibilité et un allèle de neutralité vis-à-vis du diabète insulino-dépendant. A partir du moment où l'on sait qu'il y a un allèle de neutralité, il n'y a que peu d'importance à le déterminer plus précisément dans le cadre de l'analyse selon la présente invention.

Ces deux allèles HLA-DQB1 ont été identifiés au niveau générique, le typage HLA de ce premier échantillon est en fait :
- HLA-DQB1*02, et
- HLA-DQB1*0501.

### IV - Autres approches non limitatives pour améliorer de l'invention :

### 1°) Utilisation d'amplicons PCR avec amorces biotinylées :

### A - Amorces biotinylées :

Il est également possible d'exploiter le jeu de sondes spécifiques de capture utilisé pour le dispositif d'analyse de la prédisposition génétique d'un patient au diabète insulino-dépendant (Chapitre III, 1°)), avec des amplicons PCR biotinylés par incorporation d'amorces biotinylées en 5', voir le tableau 9 ci-dessous.

**Tableau 9 : Amorces biotinylées pour amplifier le gène HLA-DQB1**

| N° de séquence | Séquence (5' > 3') | Appellation scientifique |
|---|---|---|
| Biotine-SEQ ID NO 1 | Biotine-CATGTGCTACTTCACCAACGG | DQBAMP-A-Biotine |
| Biotine-SEQ ID NO 2 | Biotine-CTGGTAGTTGTGTCTGCACAC | DQBAMP-B-Biotme |

Si les conditions d'amplification sont identiques à celles décrites plus haut, le mode opératoire est lui différent.

### B - Mode opératoire avec des amplicons biotinylés :

Premièrement, la **préparation des cibles** consiste à :
- transférer la totalité des amplicons (50 µl) dans un microtube de 1,5 ml,
- ajouter 5 µl de réactif NaOH à 2N,
- bien homogénéiser,
- incuber pendant 5 minutes à une température comprise entre 18 à 25°C,
- ajouter 1 ml de tampon d'hybridation, dont la constitution a déjà été précisée ci-dessus, et
- bien homogénéiser.

Le tampon d'hybridation est constitué de :
- 0,1 M phosphate de sodium pH 6,8,
- 0,5 M Nacl, 2% (p/v) polyethylene glycol 4000,
- 0,65% (p/v) tween 20, 0,1% (p/y) gélatine,
- 0,14g/L d'ADN soniqué de sperme de saumon,
- 0,2 g/L de BND (biocide ou Bromo-Nitro-Dioxane), et
- 0,01 g/L de ciprofloxacine.

La sonde de détection SEQ ID NO 14 (D4-POD) est diluée en tampon phosphate 5 mM à un pH de 7,0, en présence de 0,5% (p/v) albumine sérique bovine et 0,5% (p/v) phénol.

Deuxièmement, l'**hybridation des cibles** est réalisée de la manière suivante:
- répartir 100 µl du mélange, dans chacun des huit puits de la barrette R1,
- recouvrir d'une feuille autocollante, et
- incuber pendant 1 heure à 37°C (37 ± 1°C).

Troisièmement, le **lavage** qui permet d'éliminer les cibles éventuelles qui ne se sont pas hybridées, et consiste à laver trois fois avec 500 µl de tampon de lavage Color 0, dilué au 1/20 en H₂O.

Quatrièmement, la **révélation** a pour objet de déterminer quelles sondes de capture ont hybridées un oligonucléotide cible. Elle est réalisée de la façon suivante :
- ajouter 100 µl de solution de conjugué Streptavidine couplée à la péroxydase (SPOD, Roche, réf. 1089153) dilué au 1/10.000 en tampon PBS avec 0,1% Tween 20, 0,02% Albumine Sérique Bovine, à un pH de 7,2,
- incuber pendant 30 minutes à une température comprise entre 18 et 25°C, à l'obscurité,
- laver trois fois avec 500 µl de tampon de lavage - Color 0, dilué au 1/20^{ème} en H₂O
- ajouter 100 µl de substrat préparé extemporanément - 1 pastille de Color 1 dans 5 ml de Color 2, dans chacun des puits
- incuber pendant 20 minutes à une température comprise entre 18 et 25°C, à l'obscurité,
- ajouter 50 µl de Color 3, et
- lire l'absorbance à 492 nm.

Les « Color 0 », « Color 1 », « Color 2 » et « Color 3 » sont identiques à ceux décrits dans la paragraphe précédent.

Cinquièmement, **l'interprétation** permet en fonction la révélation qui a été établie au chapitre précédent de déterminer la réelle prédisposition génétique d'un patient au diabète insulino-dépendant. Cette vérification propose de :
- d'une part, vérifier la positivité du contrôle positif SEQ ID NO 3 (C+), où la Densité Optique (D.O.) doit être supérieure à 0,8, et vérifier la négativité du contrôle négatif SEQ ID NO 4 (C-), où la D.O. doit être inférieure à 0,05),
- d'autre part, déterminer le génotype correspondant aux deux allèles identifiés à l'aide des sondes SEQ ID NO 5 à SEQ ID NO 13.

Ce protocole est plus long et un peu plus complexe, puisqu'il y a une étape supplémentaire, mais des signaux plus intenses sont observés pour les sondes faibles, notamment pour SEQ ID NO 6.

Le tableau 10 ci-dessous montre bien d'ailleurs les différences de résultats obtenus entre les amplicons biotinylés et non biotinylés.

**Figure 10 : Valeurs de DO (×1000) obtenues avec une lignée hétérozygote HLA-DQB1*0302 / 0605**

| Sondes | Amplicons non biotinylés | Aplicons biotinylés |
|---|---|---|
| SEQ ID NO 3 (C +) | > 2500 | > 2500 |
| SEQ ID NO 4 (C -) | 0 | 0 |
| SEQ ID NO 5 (S) | 1 | 14 |
| SEQ ID NO 6 (S) | 134 | 324 |
| SEQ ID NO 7 (P) | 15 | 54 |
| SEQ ID NO 8 (P) | 11 | 34 |
| SEQ ID NO 9 (P) | 19 | 35 |
| SEQ ID NO 10 à 13 (N) | 603 | 1322 |

A noter qu'il n'y a plus d'utilisation, pour les amplicons biotinylés, de bras aminé en 5', qui autorise la fixation des sondes de détection en fond de plaque, comme précédemment décrit, puisque l'on utilise de la Streptavidine associée à la Péroxydase, qui va pouvoir se fixer à la Biotine présente sur les amplicons qui sont capturés.

### 2°) Utilisation de sondes de capture absorbées sur des billes :

Afin d'augmenter les signaux spécifiques pour le puits où est présent, par exemple, la sonde de séquence SEQ ID NO 6, il est possible d'utiliser un revêtement, aussi appelé « coating », avec une suspension de 100 µl de billes de polystyrène de 0,1 mm de diamètre. Ces billes proviennent de la société Polysciences (Warrington (PA) aux Etats-Unis d'Amérique - réf. : 00876), elles sont recouvertes par des molécules d'avidine fabriquées par la société Sigma (Saint-Louis (MO) aux Etats-Unis d'Amérique - réf. : A9275), sensibilisées par l'oligonucléotide spécifique de capture SEQ ID NO 6, préparé sous forme biotinylée en 5'. Dans ce cas, du fait de la présence de la biotine, l'oligonuclëotide spécifique de capture SEQ ID NO6 ne comporte pas de bras de liaison.

### A-Préparation des billes :

### 1 - Préparation des billes revêtues d'avidine :

Il convient de :
- diluer 2,5 µl de solution d'avidine à la concentration de 5 mg/ml, dans 100 µl de tampon de borate 50 mM à pH de 9,3,
- ajouter 7,4 µl de suspension de billes, et
- incuber pendant 30 minutes à la température de 37C.

### 2 - Préparation des billes revêtues d'avidine et portant des sondes de capture:

Il faut en plus :
- ajouter 5.10¹⁵ molécules de sonde SEQ ID NO 6 biotinylée, et
- incuber pendant 30 minutes à la température de 37°C.

### B - Revêtement avec les billes revêtues d'avidine et portant des sondes de capture :

Une dilution de la suspension de billes sensibilisées, au 1/10 en tampon derevêtement (3X PBS), est ensuite utilisée, à raison de 100 µl par puits. Les conditions de revêtement utilisées pour des sondes aminées, c'est-à-dire 2 heures à une température de 37°C ou 15 heures à une température comprise entre 18 et 25°C, restent inchangées par rapport à la fixation en fond de puits.

Ce procédé permet également d'augmenter les signaux dans certains puits, par exemple, où est présent la sonde de séquence SEQ ID NO 6, et ainsi de faciliter l'analyse du puits concerné. D'ailleurs les valeurs de D.O. (x 1000) obtenues pour la sonde SEQ ID NO 6, avec une lignée homozygote HLA-DQB1*0302, donne les résultats suivants :
- sans billes : 172, et
- avec billes : 689.

### 3°) Utilisation d'un seul puits pour les sondes de captures spécifiques des allèles de neutralité vis-à-vis du diabète insulino-dépendant :

Mais dans le cas de l'utilisation de la Péroxydase pour effectuer la révélation des hydridation d'oligonucléotides cibles sur les oligonucléotides de capture, le puits unique peut être utilisé pour la déterminationde « neutralité des allèles » vis-à-vis du diabète insulino-dépendant. Il comprend alors un ensemble de quatre sondes spécifiques de capture (SEQ ID NO 10 à 13) qui chacune individuellement, possède une bonne spécificité.

Comme évoqué précédemment, le développement d'un procédé de dépôts multiples au fond d'un même puits d'une plaque de microtitration, par exemple en format quatre-vingt-seize (96) puits, permet de réaliser le dépôt séparé pour les quatre sondes ci-dessus mentionnées dans un seul et unique puits.

### 4°) Utilisation d'un seul puits pour les sondes de captures spécifiques de l'ensemble des allèles concernés par le diabète insulino-dépendant :

Le développement d'un procédé de dépôts multiples au fond d'un puits, d'une plaque de microtitration, permet le dépôt des onze (11) sondes non spécifiques (SEQ ID NO 3 et 4) et spécifiques (SEQ ID NO 5 à 13) de capture, décrites précédemment dans les tableaux 3 et 4, dans un même puits. La réalisation de l'étape d'hybridation des amplicons en un puits unique, apporte différents avantages :
- avantage de sécurité pour le test, puisque les onze (11) sondes rencontrent simultanément tous les amplicons de l'échantillon avec absence de risque de faux négatif,
- avantage de sensibilité pour le test, car les onze (11) sondes rencontrent la totalité des amplicons de l'échantillon, et
- avantage de pouvoir réaliser des grandes séries de test avec la possibilité de traiter quatre-vingt-seize (96) échantillons par plaque, ce qui est particulièrement intéressant pour les applications de dépistage.

Comme évoqué précédemment, cette approche nécessite par contre une révélation ponctuelle des hybridations spécifiques au fond des puits. A titre d'exemple, il est possible d'utiliser la fluorescence pour la détection d'amplicons ayant incorporer une molécule fluorescente ou révélablé à l'aide d'un marqueur fluorescent.

Des essais de faisabilité d'exploitation de plusieurs dépôts en un même puits, ont également été réalisés, avec une révélation colorimétrique.

### A - Dépôts :

Les essais avec quatre (4) dépôts manuels, de sondes spécifiques de capture (SEQ ID NO 10 à 13, en version aminée), avec des dépôts de 1 et 5 µl, à 400-1000 pmoles/ml en tampon de revêtement ou coating (PBS 3X). On incube entre 15 minutes et 2 heures à la température de 37°C, ou pendant 15 heures à une température comprise entre 18 et 25°C.

### B - Lavage:

ll s'effectue par Color 0 dilué.

### C - Préparation des cibles :

Les amplicons PCR sont préparés comme décrit au paragraphe précédent (III - 1°) - C).

Dans le tube d'amplification, on effectue les étapes suivantes :
- dénaturation des 50 µl d'amplicons par addition de 1 µl de 2N NaOH,
- incubation pendant 5 minutes à une température comprise entre 18 et 25°C,
- addition de 40 µl de tampon d'hybridation (SSPE 15X, 2% PEG 4000,1,5% tween 20, 0,22% gélatine, 0,032% ADN soniqué, biocides),
- addition de 6 µl de solution de conjugué D4-POD (50 pmoles/ml ; tampon phosphate 5 mM pH 7.0 - 0,5% (p/v) ASB - 0,5% (p/v) phénol).

### D-Hybridation :

On transfère la totalité du mélange réactionnel dans un puits sensibilisé avec les quatre (4) dépôts et on incube pendant une heure à la température de 37°C à ± 1 °C.

### E - Lavage :

Il s'effectue par Color 0 dilué.

### F - Révélation :

On effectue les étapes successives suivantes :
- addition de 100 µl de solution de substrat (Color 1 dilué en Color 2),
- développement sans perturbation, pendant 20 minutes, à une température comprise entre 18 et 25°C, à l'obscurité,
- observer l'apparition de coloration à l'endroit de la sonde positive,
- pour quantifier le signal, addition de 50 µl de Color 3,
- homogénéiser en agitant doucement, et
- lire à 492 nm.

### Références bibliographiques

Benjamin, 1990 :
   Benjamin R, Parham P, Immunology Today, 1990, 11, 137-142, Guilt by association : HLA-B27 and ankylosing spondylitis
Brewerton, 1973 :
   Brewerton DA, Caffrey M, Hart FD et al, Lancet, 1973, 1, 904-907. Ankylosing spondylitis and HL-A27.
Gregersen, 1986 :
   Gregersen PK, Shen M, Song QL, Merryman P, Degar S, Seki T, Maccari J, Goldberg D, Murphy H, Schwenzer J, Wang CY, Winchester RJ, Nepom GT, Silver J, Proc. Natl. Acad . Sci. USA, 1986, 83, 2642-2646. Molecular diversity of HLA-DR4 haplotypes.
Gregersen. 1987 :
   Gregersen PK, Silver J, Winchester RJ, Arthritis Rheum., 1987, 30, 1205-1213. The shared epitope hypothesis. An approach to understanding the molecular genetics of susceptibility to rheumatoid arthritis.
Hiraiwa. 1990:
   Hiraiwa A, Yamanaka K, Kwok WW, Mickelson EM, Masewicz S, Hansen JA, Radka SF, Nepom GT, Proc. Natl. Acad. Sci. USA, 1990, 87, 8051-8055. Structural requirements for recognition of the HLA-Dw14 class II epitope : a key HLA déterminant associated with rheumatoid arthritis.
Khan, 1979:
   Khan MA, Kushner I, Ballou SP et al, Lancet, 1979, 1, 921-922, Familial rheumatoid arthritis and HLA-DRW4.
Komulainen, 1999
   Komulainen J, Kulmala P, Savola K,Lounamaa R, Ilonen J, Reijonen H, Knip M, Akerblom HK; Diabetes Care 1999, vol 22, n°12, 1950-1955
Kulmala, 2000
   Kulmala P, Savola K, Reijonen H, Veijola R, Vahasalo P, Karjalainen J, Thuomiletho-Wolf E, Ilonen J, Tuomiletho J, Akerblom HK, Knip M ; Diabetes 2000, vol 49, 48-58
Lawrence, 1970 :
   Lawrence J, Ann. Rheum. Dis., 1970, 29, 357-379
Nepom, 1989 :
   Nepom GT, Byers P, Seyfried C, Healey LA, Wilske KR, Stage D, Nepom BS, Arthritis Rheum. 1989, 32, 15-21. HLA genes associated with rheumatoid arthritis. Identification of susceptibility alleles using specific oligonucleotide probes.
Nepom, 1991 :
   Nepom GT, Erlich H, Annu. Rev. Immunol., 1991,9,493-525. MHC class-II molecules and autoimmunity.
Schlosstein, 1973 :
   Schlosstein L, Terasaki PI, Bluestone R, Pearson CM, N. Engl. J. Med., 1973, 288, 704-706. High association of a HL-A antigen, w27, with ankylosing spondylitis
Stastny, 1978 :
   Stastny P, N. Engl. J. Med., 1978, 298, 869-871. Association of the B-cell alloantigen DRw4 with rheumatoid arthritis.
Stastny. 1983 :
   Stastny P, Ball EJ, Dry PJ, Nunez G, Immunol. Rev., 1983, 70, 113-154. The human immune response region (HLA-D) and disease susceptibility.
Stastny, 1988 :
   Stastny P, Ball E, Kahn M, Olsen N, Pincus T, Gao X, Br. J. Rheumatol., 1988, 27, 132-138. HLA-DR4 and other genetic markers in rheumatoid arthritis.
Todd. 1988 :
   Todd JA, Acha-Orbea H, Bell JI, Chao N, Fronek Z, Jacob CO, McDermott M, Sinha AA, Timmerman L, McDevitt HO, Science, 1988, 240, 1003-1009
Wordsworth. 1989:
   Wordsworth BP, Lanchbury JS, Sakkas LI, Welsh KI, Panayi GS, Bell JI, Proc. Nati. Acad. Sci. USA, 1989, 86, 10049-10053

## Revendications

1. Procédé d'analyse de la prédisposition génétique d'un patient à une maladie auto-immune, consistant à mettre un échantillon liquide contenant au moins un type d'amplicons, issus de l'amplification d'au moins une région polymorphe d'intérêt en rapport avec la maladie recherchée, à savoir le diabète insulino-dépendant, en présence simultanément de sondes choisies de la façon suivante :
• au moins une sonde spécifique des allèles HLA-DQB1*0201 et HLA-DQB1*0202 et HLA-DQB1*0302 pour la susceptibilité,
• au moins une sonde spécifique des allèles HLA-DQB1*0301, HLA-DQB1*0602 et HLA-DQB1*0603 pour la protection, et
• au moins une sonde spécifique des autres allèles HLA-DQB1* associés au diabète insulino-dépendant pour la neutralité.

2. Procédé selon la revendication 1, **caractérisé en ce que** les sondes sont choisies de la façon suivante :
• au moins une sonde spécifique des allèles HLA-DQB1*0201, HLA-DQB1*0202, HLA-DQB1*0203, HLA-DQB1*0302, HLA-DQB1*0304, HLA-DQB1*0305, HLA-DQB1*0307 et HLA-DQB1*0308 pour la susceptibilité,
• au moins une sonde spécifique des allèles HLA-DQB1*03011, HLA-DQB1*03012, HLA-DQB1*03032, HLA-DQB1*03033, HLA-DQB1*0304, HLA-DQB1*0306, HLA-DQB1*0308, HLA-DQB1*0309, HLA-DQB1*0310, HLA-DQB1*0602, HLA-DQB1*0603, HLA-DQB1*0608, HLA-DQB1*0610, HLA-DQB1*06111, HLA-DQB1*06112, HLA-DQB1*0612, HLA-DQB1*0613, HLA-DQB1*0614 et HLA-DQB1*0616 pour la protection, et
• au moins une sonde spécifique des allèles HLA-DQ131*0306, HLA-DQB1*0401, HLA-DQB1*0402, HLA-DQB1*05011, HLA-DQB1*05012, HLA-DQB1*0502, HLA-DQB1*05031, HLA-DQB1*05032, HLA-DQB1*06011, HLA-DQB1*06412, HLA-DQB1*06013, HLA-DQB1*06051, HLA-DQB1*06052, HLA-DQB1*0606, HLA-DQB1*0609, HLA-DQB1*06112 et HLA-DQB1*0612 pour la neutralité.

3. Procédé, selon la revendication 2, **caractérisé en ce que** les sondes utilisées pour détecter la susceptibilité vis-à-vis du diabète insulino-dépendant sont définies comme suit :
• une sonde spécifique des allèles HLA-DQB1*0201, HLA-DQB1*0202 et HLA-DQB1*0203, et
• une sonde spécifique des allèles HLA-DQB1*0302, HLA-DQB1*0304, HLA-DQB1*0305, HLA-DQB1*0307 et HLA-DQB1*0308.

4. Procédé, selon la revendication 3, **caractérisé en ce que** les sondes utilisées pour détecter la susceptibilité vis-à-vis du diabète insulino-dépendant sont constituées d'au moins dix (10) nucléotides successifs pris dans les séquences suivantes :
• SEQ ID NO 5 (TCTTgTgAgCAgAAgC), et
• SEQ ID NO 6 (CCgCCTgCCgCCgA).

5. Procédé, selon la revendication 2, **caractérisé en ce que** les sondes utilisées pour détecter la protection vis-à-vis du diabète insulino-dépendant sont définies comme suit :
• une sonde spécifique des allèles MLA-DQB1*03011, HLA-DQB1*03012, HLA-DQB1*0304 et HLA-DQB1*0309,
• une sonde spécifique des allèles HLA-DQB1*03011, HLA-DQB1*03012, HLA-DQB1*03032, HLA-DQB1*03033, HLA-DQB1*0306, HLA-DQB1*0309 et DQB1*0310, et
• une sonde spécifique des allèles HLA-DQB1*0308, HLA-DQB1*0602, HLA-DQB1*0603, HLA-DQB1*0608, HLA-DQB1*0610, HLA-DQB1*06111, HLA-DQB1*06112, HLA-DQB1*0612, HLA-DQB1*0613, HLA-DQB1*0614 et HLA-DQB1*0616.

6. Procédé, selon la revendication 5, **caractérisé en ce que** les sondes utilisées pour détecter la protection vis-à-vis du diabète insulino-dépendant sont constituées d'au moins dix (10) nucléotides successifs pris dans les séquences suivants :
• SEQ ID NO 7 (AggggACCCgggCggA),
• SEQ ID NO 8 (gACgTggAggTgTACC), et
• SEQ ID NO 9 (gCCgCCTgACgCCg).

7. Procédé, selon la revendication 2, **caractérisé en ce que** les sondes utilisées pour détecter la neutralité vis-à-vis du diabète insulino-dépendant sont définies comme suit :
• une sonde spécifique des allèles HLA-DQB1*0306, HLA-DQB1*0401 et HLA-DQB1*0402,
• une sonde spécifique des allèles HLA-DQBI*05011, HLA-DQB1*05012, HLA-DQB1*0502, HLA-DQB1*05031 et HLA-DQB1*05032,
• une sonde spécifique des allèles HLA-DQB1*06011, HLA-DQB1*06012 et HLA-DQB1*06013, et
• une sonde spécifique des allèles HLA-DQB1*06051, HLA-DQB1*06052, HLA-DQB1*0606, HLA-DQB1*0609, HLA-DQB1*06112 et HLA-DQB1*0612.

8. Procédé, selon la revendication 7, **caractérisé en ce que** les sondes utilisées pour détecter la neutralité vis-à-vis du diabète insulino-dépendant sont constituées d'au moins dix (10) nucléotides successifs pris dans les séquences suivants :
• SEQ ID NO 10 (ggggCCCgggCgTC),
• SEQ ID NO 11 (AggAggACgTgCgC),
• SEQ ID NO 12 (TCTTgTAACCAgATAC), et
• SEQ ID NO 13 (ggTggACACCgTATgCAg).

9. Procédé, selon l'une quelconque des revendications précédentes, **caractérisé en ce qu'**au moins une sonde de contrôle positif, capable de s'hybrider avec l'ensemble des gènes HLA-DQB1, est utilisée pour permettre la détection de tous les allèles HLA-DQB1.

10. Procédé, selon l'une quelconque des revendications 4, 6, 8 ou 9, **caractérisé en ce qu'**au plus 38,89 %, préférentiellement au plus 20 %, des bases d'une même sonde est remplacée par au moins une base analogue, telle que l'inosine.

11. Procédé, selon l'une quelconque des revendications 1 à 10, **caractérisé en ce que**, préalablement, au moins une amplification du gène HLA-DQB1 est effectuée.

12. Procédé, selon la revendication 11, **caractérisé en ce que**, les amorces d'amplification sont biotinylées, de sorte que les amplicons sont également biotinylés.

13. Procédé, selon l'une quelconque des revendications 11 ou 12, **caractérisé en ce que**, préalablement à l'amplification, l'échantillon biologique prélevé, préférentiellement sous forme de tache de sang séché, est traité en vue de l'extraction des acides nucléiques.

14. Procédé, selon la revendication 13, **caractérisé en ce que** l'extraction des acides nucléiques s'effectue dans un mélange réactionnel contenant déjà les désoxyribonucléotides triphosphates (dNTP), qui seront ensuite utilisés lors de l'amplification, et ce préalablement à l'incubation.

15. Procédé, selon la revendication 14, **caractérisé en ce qu'**après l'incubation, on ajoute dans le mélange réactionnel, des désoxyribonucléotides triphosphates (dNTP), qui seront également utilisés lors de l'amplification ultérieure.

16. Dispositif permettant la mise en oeuvre d'un procédé, selon l'une quelconque des revendications 1 à 15, **caractérisé en ce que** chaque type de sondes DQB1 spécifiques de la susceptibilité, de la protection et de la neutralité est fixé dans un compartiment indépendant

17. Dispositif permettant la mise en oeuvre d'un procédé selon l'une quelconque des revendications 1 à 15, **caractérisé en ce que** chaque type de sondes utilisées pour détecter la susceptibilité ou la protection vis-à-vis du diabète insulino-dépendant est fixé dans un compartiment indépendant, indépendamment des autres sondes utilisées pour détecter cette susceptibilité ou cette protection, et que toute ou partie des différents types de sondes utilisées pour détecter la neutralité vis-à-vis du diabète insulino-dépendant est fixée dans au moins un puits d'une plaque de microtitration.

18. Dispositif permettant la mise en oeuvre d'un procédé selon l'une quelconque des revendications 1 à 15, **caractérisé en ce que** l'ensemble des types de sondes utilisées pour détecter la susceptibilité ou la protection ou la neutralité vis-à-vis du diabète insulino-dépendant est fixé dans un seul et même compartiment.

19. Dispositif selon l'une quelconque des revendications 16 à 18, dans lequel les sondes sont fixées au fond d'un puits de plaque de microtitration ou sur une bille par l'intermédiaire d'un bras aminé ou de biotine situé(e) en position 5' des sondes.

## Patentansprüche

1. Ein Verfahren zur Analyse der genetischen Prädisposition eines Patienten für eine Autoimmunerkrankung, das aus dem gleichzeitigen Zusammenbringen einer flüssigen Probe, die mindestens eine Art von Amplikonen beinhaltet, die aus der Amplifikation von mindestens einer polymorphen Region von Interesse mit Bezug auf die gesuchte Krankheit, d. h. insulinabhängiger Diabetes mellitus, entstehen, mit Gensonden besteht, die auf folgende Weise gewählt werden:
• mindestens eine Gensonde, die für die Allele HLA-DQB1*0201 und HLA-DQB1*0202 und HLA-DQB1*0302 für die Anfälligkeit spezifisch ist,
• mindestens eine Gensonde, die für die Allele HLA-DQB1*0301, HLA-DQB1*0602 und HLA-DQB1*0603 für den Schutz spezifisch ist, und
• mindestens eine Gensonde, die für die anderen HLA-DQB1*-Allele, die mit insulinabhängigem Diabetes mellitus verbunden sind, für die Neutralität spezifisch ist.

2. Verfahren gemäß Anspruch 1, **dadurch gekennzeichnet, dass** die Gensonden auf folgende Weise gewählt werden:
• mindestens eine Gensonde, die für die Allele HLA-DQB1*0201, HLA-DQB1*0202, HLA-DQB1*0203, HLA-DQB1*0302, HLA-DQB1*0304, HLA-DQB1*0305, HLA-DQB1*0307 und HLA-DQB1*0308 für die Anfälligkeit spezifisch ist,
• mindestens eine Gensonde, die für die Allele HLA-DQB1*03011, HLA-DQB1*03012, HLA-DQB1*03032, HLA-DQB1*03033, HLA-DQB1*0304, HLA-DQB1*0306, HLA-DQB1*0308, HLA-DQB1*0309, HLA-D0B1*0310, HLA-DQB1*0602, HLA-DQB1*0603, HLA-DQB1*0608, HLA-DQB1*0610, HLA-DQB1*06111, HLA-DQB1*06112, HLA-DQB1*0612, HLA-DQB1*0613, HLA-DQB1*0614 und HLA-DQB1*0616 für den Schutz spezifisch ist, und
• mindestens eine Gensonde, die für die Allele HLA-DQB1*0306, HLA-DQB1*0401, HLA-DQB1 *0402, HLA-DQB1*05011, HLA-DQB1*05012, HLA-DQB1*0502, HLA-DQB1*05031, HLA-DQB1*05032, HLA-DQB1*06011, HLA-DQB1*06012, HLA-DQB1*06013, HLA-DQB1*06051, HLA-DQB1*06052, HLA-DQB1*0606, HLA-DQB1*0609, HLA-DQB1*08112 und HLA-DQB1*0612 für die Neutralität spezifisch ist.

3. Verfahren gemäß Anspruch 2, **dadurch gekennzeichnet, dass** die Gensonden, die zum Nachweisen der Anfälligkeit gegenüber insulinabhängigem Diabetes mellitus verwendet werden, wie folgt definiert sind:
• eine Gensonde, die für die Allele HLA-DQB1*0201, HLA-DQB1*0202 und HLA-DQB1*0203 spezifisch ist, und
• eine Gensonde, die für die Allele HLA-DQB1*0302, HLA-DQB1*0304, HLA-DQB1*0305, HLA-DQB1*0307 und HLA-DQB1*0308 spezifisch ist.

4. Verfahren gemäß Anspruch 3, **dadurch gekennzeichnet, dass** die Gensonden, die zum Nachweisen der Anfälligkeit gegenüber insulinabhängigem Diabetes mellitus verwendet werden, aus mindestens zehn (10) aufeinanderfolgenden Nukleotiden aus den folgenden Sequenzen bestehen:
• SEQ ID NR. 5 (TCTTgTgAgCAgAAgC), und
• SEQ ID NR. 6 (CCgCCTgCCgCCgA).

5. Verfahren gemäß Anspruch 2, **dadurch gekennzeichnet, dass** die Gensonden, die zum Nachweisen des Schutzes gegenüber insulinabhängigem Diabetes mellitus verwendet werden, wie folgt definiert sind:
• eine Gensonde, die für die Allele HLA-DQB1*03011,HLA-DQB1*03012, HLA-DQB1*0304 und HLA-DQB1*0309, spezifisch ist, und
• eine Gensonde, die für die Allele HLA-DQB1*03011, HLA-DQB1*03012, HLA DQB1*03032, HLA-DQB1*03033, HLA-DQB1*0306, HLA-DQB1*0309 und HLA-DQB1*0310 spezifisch ist, und
• eine Gensonde, die für die Allele HLA-DQB1*0308, HLA-DQB1*0602, HLA-DQB1*0603, HLA-DQB1*0608, HLA-DQB1*0610, HLA-DQB1*06111, HLA-DQB1*06112, HLA-DQB1*0612, HLA-DQB1*0613, HLA-DQB1*0614 und HLA-DQB1*0616 spezifisch ist.

6. Verfahren gemäß Anspruch 5, **dadurch gekennzeichnet, dass** die Gensonden, die zum Nachweisen des Schutzes gegenüber insulinabhängigem Diabetes mellitus verwendet werden, aus mindestens zehn (10) aufeinanderfolgenden Nukleotiden aus den folgenden Sequenzen bestehen:
• SEQ ID NR. 7 (AggggACCCgggCggA),
• SEQ ID NR. 8 (gACgTggAggTgTACC), und
• SEQ ID NR. 9 (gCCgCCTgACgCCg).

7. Verfahren gemäß Anspruch 2, **dadurch gekennzeichnet, dass** die Gensonden, die zum Nachweisen der Neutralität gegenüber insulinabhängigem Diabetes mellitus verwendet werden, wie folgt definiert sind:
• eine Gensonde, die für die Allele HLA-DQB1*0306, HLA-DQB1*0401 und HLA-DQB1*0402 spezifisch ist,
• eine Gensonde, die für die Allele HLA-DQB1*05011, HLA-DQB1*05012, HLA-DQB1*0502, HLA-DQB1*05031 und HLA-DQB1*05032 spezifisch ist,
• eine Gensonde, die für die Allele HLA-DQB1*06011, HLA-DQB1*06012 und HLA-DQB1*06013 spezifisch ist, und
• eine Gensonde, die für die Allele HLA-DQB1*06051, HLA-DQB1*06052, HLA-DQB1*0606, HLA-DQB1*0609, HLA-DQB1*06112 und HLA-DQB1*0612 spezifisch ist.

8. Verfahren gemäß Anspruch 7, **dadurch gekennzeichnet, dass** die Gensonden, die zum Nachweisen der Neutralität gegenüber insulinabhängigem Diabetes mellitus verwendet werden, aus mindestens zehn (10) aufeinanderfolgenden Nukleotiden aus den folgenden Sequenzen bestehen:
• SEQ ID NR. 10 (ggggCCCgggCgTC),
• SEQ ID NR. 11 (AggAggACgTgCgC),
• SEQ ID NR. 12 (TCTTgTAACCAgATAC), und
• SEQ ID NR.13 (ggTggACACCgTATgCAg).

9. Verfahren gemäß einem der vorhergehenden Ansprüche, **dadurch gekennzeichnet, dass** mindestens eine Gensonde positiver Kontrolle, die mit allen der HLA-DQB1-Gene hybridisieren kann, verwendet wird, um das Nachweisen von allen der Allele HLA-DQB1 zu ermöglichen.

10. Verfahren gemäß einem der Ansprüche 4, 6, 8 oder 9, **dadurch gekennzeichnet, dass** höchstens 38,89 %, vorzugsweise höchstens 20 %, der Basen einer gleichen Gensonde durch mindestens eine analoge Base, wie etwa Inosin, ersetzt werden.

11. Verfahren gemäß einem der Ansprüche 1 bis 10, **dadurch gekennzeichnet, dass** vorab mindestens eine Amplifikation des Genes HLA-DQB1 durchgeführt wird.

12. Verfahren gemäß Anspruch 11, **dadurch gekennzeichnet, dass** die Amplifikationsprimer biotinyliert werden, so dass die Amplikonen auch biotinyliert werden.

13. Verfahren gemäß Anspruch 11 oder 12, **dadurch gekennzeichnet, dass** die entnommene biologische Probe, vorzugsweise in Form eines getrockneten Blutflecks, vor der Ampüfikation behandelt wird, mit dem Ziel, Nukleinsäuren zu extrahieren.

14. Verfahren gemäß Anspruch 13, **dadurch gekennzeichnet, dass** das Extrahieren von Nukleinsäuren in einer Reaktionsmischung durchgeführt wird, die schon Desoxyribonukleotid-Triphosphate (dNTP) beinhaltet, die dann bei der Amplifikation und vor der Inkubation verwendet werden.

15. Verfahren gemäß Anspruch 14, **dadurch gekennzeichnet, dass** nach der Inkubation Desoxyribonukleotid-Triphosphate (dNTP), die auch bei der nachfolgenden Amplifikation verwendet werden, zu der Reaktionsmischung hinzugegeben werden.

16. Eine Vorrichtung, die die Ausführung eines Verfahrens gemäß einem der Ansprüche 1 bis 15 ermöglicht, **dadurch gekennzeichnet, dass** jede Art von DQB1-Gensonden, die für die Anfälligkeit, den Schutz und die Neutralität spezifisch sind, in einer unabhängigen Kammer angeordnet wird.

17. Vorrichtung, die die Ausführung eines Verfahrens gemäß einem der Ansprüche 1 bis 15 ermöglicht, **dadurch gekennzeichnet, dass** jede Art von Gensonden, die zum Nachweisen der Anfälligkeit oder des Schutzes gegenüber insulinabhängigem Diabetes mellitus verwendet werden, in einer unabhängigen Kammer, unabhängig von den anderen Gensonden, die zum Nachweisen der Anfälligkeit oder des Schutzes verwendet werden, angeordnet ist, und **dadurch**, **dass** alle oder ein Teil der unterschiedlichen Arten von Gensonden, die zum Nachweisen der Neutralität gegenüber insulinabhängigem Diabetes mellitus verwendet werden, in mindestens einem Well einer Mikrotiterplatte angeordnet werden/wird.

18. Vorrichtung, die die Ausführung eines Verfahrens gemäß einem der Ansprüche 1 bis 15 ermöglicht, **dadurch gekennzeichnet, dass** die Gesamtheit der Arten von Gensonden, die zum Nachweisen der Anfälligkeit oder des Schutzes oder der Neutralität gegenüber insulinabhängigem Diabetes mellitus verwendet werden, in derselben Kammer angeordnet wird.

19. Vorrichtung gemäß einem der Ansprüche 16 bis 18, wobei die Gensonden auf einen Wellboden einer Mikrotiterplatte oder auf einer Perle mittels eines Amino-Spacers oder Biotin, der/das sich bei Position 5' der Gensonden befindet, angeordnet werden.

## Claims

1. A method for analysing a patient's genetic predisposition to an auto-immune disease, consisting in bringing a liquid sample containing at least one type of amplicons, which are the products of the amplification of at least one polymorphic region of interest in relation to the disease investigated, i.e. insulin-dependent diabetes mellitus, simultaneously in the presence of probes chosen in the following manner:
• at least one probe specific to the alleles HLA-DQB1*0201 and HLA-DQB1*0202 and HLA-DQB1*0302 for susceptibility,
• at least one probe specific to the alleles HLA-DQB1*0301, HLA-DQB1*0602 and HLA-DQB1*0603 for protection, and
• at least one probe specific to the other HLA-DQB1* alleles associated with insulin-dependent diabetes mellitus for neutrality.

2. The method according to Claim 1, **characterised in that** the probes are chosen in the following manner:
• at least one probe specific to the alleles HLA-DQB1*0201 , HLA-DQB1*0202, HLA-DQB1*0203, HLA-DQB1*0302, HLA-DQB1*0304, HLA-DQB1 *0305, HLA-DQB1*0307 and HLA-DQB1*0308 for susceptibility,
• at least one probe specific to the alleles HLA-DQB1*03011, HLA-DQB1*03012, HLA-DQB1*03032, HLA-DQB1*03033, HLA-DQB1*0304, HLA-DQB1*0306, HLA-DQB1*0308, HLA-DQB1*0309, HLA-DQB1*0310, HLA-DQB1*0602, HLA-DQB1*0603, HLA-DQB1*0608, HLA-DQB1*0610, HLA-DQB1*06111, HLA-DQB1*06112, HLA-DQB1*0612, HLA-DQB1*0613, HLA-DQB1*0614 and HLA-DQB1*0616 for protection, and
• at least one probe specific to the alleles HLA-DQB1*0306, HLA-DQB1*0401, HLA-DQB1*0402, HLA-DQB1*05011, HLA-DQB1*05012, HLA-DQB1*0502, HLA-DQB1*05031, HLA-DQB1*05032, HLA-DQB1*06011, HLA-DQB1*06012, HLA-DG1B1*06013, HLA-DQB1*06051, HLA-DQB1*06052, HLA-DQB1*0606, HLA-DQB1*0609, HLA-DQB1*06112 and HLA-DQB1*0612 for neutrality.

3. The method according to Claim 2, **characterised in that** the probes used to detect susceptibility with regard to insulin-dependent diabetes mellitus are defined as follows:
• a probe specific to the alleles HLA-DQB1*0201, HLA-DQB1*0202 et HLA-DQB1*0203, and
• a probe specific to the alleles HLA-DQB1*0302, HLA-DQB1*0304, HLA-DQB1*0305, HLA-DQB1*0307 and HLA-DQB1*0308.

4. The method according to Claim 3, **characterised in that** the probes used to detect susceptibility with regard to insulin-dependent diabetes mellitus consist of at least ten (10) successive nucleotides from the following sequences:
• SEQ ID NO 5 (TCTTgTgAgCAgAAgC), and
• SEQ ID NO 6 (CCgCCTgCCgCCgA).

5. The method according to Claim 2, **characterised in that** the probes used to detect protection with regard to insulin-dependent diabetes mellitus are defined as follows:
• a probe specific to the alleles HLA-DQB1*03011, HLA-DQB1*03012, HLA-DQB1*0304 and HLA-DQB1*0309,
• a probe specific to the alleles HLA-DQB1*03011, HLA-DQB1*03012, HLA-DQB1*03032, HLA-DQB1*03033, HLA-DQB1*0306, HLA-DQB1*O309 and HLA-DQB1*0310, and
• a probe specific to the alleles HLA-DQB1*0308, HLA-DQB1*0602, HLA-DQB1*0603, HLA-DQB1*0608, HLA-DQB1*0610, HLA-DQB1*06111, HLA-DQB1*06112, HLA-DQB1*0612, HLA-DQB1*0613, HLA-DQB1*0614 and HLA-DQB1*0616.

6. The method according to Claim 5, **characterised in that** the probes used to detect protection with regard to insulin-dependent diabetes mellitus consist of at least ten (10) successive nucleotides from the following sequences:
• SEQ ID NO 7 (AggggACCCgggCggA),
• SEQ ID NO 8 (gACgTggAggTgTACC), et
• SEQ ID NO 9 (gCCgCCTgACgCCg).

7. The method according to Claim 2, **characterised in that** the probes used to detect neutrality with regard to insulin-dependent diabetes mellitus are defined as follows:
• a probe specific to the alleles HLA-DQB1*0306, HLA-DQB1*0401 and HLA-DQB1*0402,
• a probe specific to the alleles HLA-DQB1*05011, HLA-DCB1*05012, HLA-DQB1*0502, HLA-DQB1*05031 and HLA-DQB1*05032,
• a probe specific to the alleles HLA-DQB1*06011, HLA-DQB1*06012 and HLA-DQB1*06013, and
• a probe specific to the alleles HLA-DQB1*06051, HLA-DQB1*06052, HLA-DQB1*0606, HLA-DQB1*0609, HLA-DQB1*06112 and HLA-DQB1*0612.

8. The method according to Claim 7, **characterised in that** the probes used to detect neutrality with regard to insulin-dependent diabetes mellitus consist of at least ten (10) successive nucleotides from the following sequences:
• SEQ ID NO 10 (ggggCCCgggCgTC),
• SEQ ID NO 11 (AggAggACgTgCgC),
• SEQ ID NO 12 (TCTTgTAACCAgATAC), and
• SEQ ID NO 13 (ggTggACACCgTATgCAg).

9. The method according to any one of the preceding claims, **characterised in that** at least one positive control probe, capable of hybridising with all of the HLA-DQB1 genes, is used to enable the detection of all of the HLA-DQB1 alleles.

10. The method according to any one of Claims 4, 6, 8 or 9, **characterised in that** at the most 38.89%, preferably at the most 20%, of the bases of a same probe is replaced by at least one base analogue, such as inosine.

11. The method according to any one of Claims 1 to 10, **characterised in that**, beforehand, at least one amplification of the HLA-DQB1 gene is carried out.

12. The method according to Claim 11, **characterised in that** the amplification primers are biotinylated, such that the amplicons are also biotinylated.

13. The method according to any one of Claims 11 or 12, **characterised in that**, prior to amplification, the biological sample taken, preferably in the form of a stain of dried blood, is treated with a view to extracting the nucleic acids.

14. The method according to Claim 13, **characterised in that** the extraction of the nucleic acids is carried out in a reaction mixture already containing the deoxyribonucleotide triphosphates (dNTP) which will then be used during amplification, prior to incubation.

15. The method according to Claim 14, **characterised in that**, after incubation, deoxyribonucleotide triphosphates (dNTP), which will also be used during the subsequent amplification, are added into the reaction mixture.

16. A device enabling the implementation of a method, according to any one of Claims 1 to 15, **characterised in that** each type of DQB1 probes specific to susceptibility, protection and neutrality is secured in an independent compartment.

17. The device enabling the implementation of a method according to any one of Claims 1 to 15, **characterised in that** each type of probes used to detect susceptibility or protection with regard to insulin-dependent diabetes mellitus is secured in an independent compartment, independently of the other probes used to detect this susceptibility or this protection, and that all or part of the different types of probes used to detect neutrality with regard to insulin-dependent diabetes mellitus is secured in at least one well of a microtitration plate.

18. The device enabling the implementation of a method according to any one of Claims 1 to 15, **characterised in that** all of the types of probes used to detect susceptibility or protection or neutrality with regard to insulin-dependent diabetes mellitus are secured in one and the same compartment.

19. The device according to any one of Claims 16 to 18, in which the probes are secured at the bottom of a well of a microtitration plate or on a bead by means of an amine arm or biotine situated at position 5' of the probes.
